(19) Europäisches Patentamt
European Patent Office
Office européen des brevets

(11) Numéro de publication: **0 234 971**
**B1**

(12) **FASCICULE DE BREVET EUROPEEN**

(45) Date de publication du fascicule du brevet: **05.09.90**

(21) Numéro de dépôt: **87400078.9**

(22) Date de dépôt: **15.01.87**

(51) Int. Cl.$^5$: **C 07 D 471/04,**
A 61 K 31/435, A 61 K 31/50
// (C07D471/04, 221:00, 221:00)

(54) **Nouveaux amides substitues, leur preparation et les compositions pharmaceutiques qui les contiennent.**

(30) Priorité: **16.01.86 FR 8600556**

(43) Date de publication de la demande:
**02.09.87 Bulletin 87/36**

(45) Mention de la délivrance du brevet:
**05.09.90 Bulletin 90/36**

(84) Etats contractants désignés:
**AT BE CH DE ES FR GB GR IT LI LU NL SE**

(56) Documents cités:
**EP-A-0 208 621**
**FR-A-1 006 725**

(73) Titulaire: **RHONE-POULENC SANTE**
**20, avenue Raymond Aron**
**F-92160 Antony (FR)**

(72) Inventeur: **Cotrel, Claude**
**17 A, avenue du Docteur Arnold Netter**
**F-75012 Paris (FR)**
Inventeur: **Guyon, Claude**
**17 bis, avenue Henri Martin**
**F-94100 Saint-Maur-des-Fosses (FR)**
Inventeur: **Roussel, Gérard**
**20 ter, rue des Carrières**
**F-91450 Soisy-sur-Seine (FR)**
Inventeur: **Taurand, Gérard**
**2/124, allée Jean de la Bruyère**
**F-94000 Creteil (FR)**

(74) Mandataire: **Savina, Jacques et al**
**RHONE-POULENC INTERSERVICES Service Brevets Pharma 25, quai Paul Doumer**
**F-92408 Courbevoie Cédex (FR)**

**EP 0 234 971 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

**Description**

Dans la demande de brevet PCT 8400489, ont été décrits des dérivés de benzamides de formule générale:

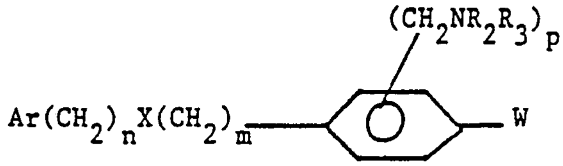

utiles comme antiarythmiques.

Dans la demande de brevet néerlandais 73 05482 et le brevet américain 3 993 656 sont décrits des dérivés de naphtyridine-1,8 de structure:

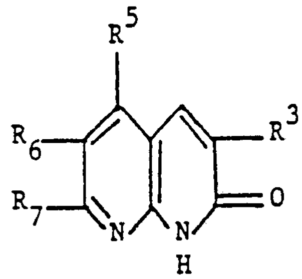

utiles comme bronchodilatateurs et vasodilatateurs périphériques ou comme agents hypotenseurs.

La présente invention concerne de nouveaux amides substitués de formule générale:

R—CONH—Het (I)

leur préparation et les compositions pharmaceutique qui les contiennent.

Dans la formule générale (I)

soit le symbole R représente un radical cycloalcoyle contenant 3 à 6 atomes de carbone, cyclohexadiényle, phényle, phényle substitué (par 1 ou 2 atomes de fluor ou par un radical hydroxy, ou substitué en position -3 ou -4 par un radical alcoyle ou alcoyloxy, ou en position -3 et -4 par un radical méthylènedioxy, ou substitué en position -2 ou -3 par un radical dialcoylamino ou en position -4 par un radical alcoylamino ou alcoyloxycarbonylamino), ou bien le symbole R représente un radical hétérocyclyle choisi parmi pyridyle-3, alcoyloxypyridyle-3, thiényle, alcoylthiényle, furyle, tétrahydropyridyle, pyridazinyle et alcoylpyridazinyle, et

le symbole Het représente un radical naphtyridine-1,8 yle-2 substitué en position -7 par un radical alcoyloxy, alcoylthio ou alcoylsulfinyle lui-même substitué par un radical alcoylamino, dialcoylamino (dont les parties alcoyle peuvent éventuellement former avec l'atome d'azote auquel elles sont rattachées, un hétérocycle à 5 ou 6 chaînons contenant éventuellement un autre hétéroatome choisi parmi l'azote, l'oxygène ou le soufre et éventuellement substitué par un radical alcoyle) ou par un radical N-alcoyl N-alcoyloxycarbonyl-amino ou dialcoylcarbamoyle, ou bien substitué en position -7 par un radical dialcoylaminovinyle,

soit le symbole R représente un radical alcoylamino-4 phényle ou alcoyloxycarbonylamino-4 phényle et

le symbole Het représente un radical naphtyridine-1,8 yle-2 substitué en position -7 par un radical alcoyloxy ou alcoylthio, étant entendu que les radicaux ou portions alcoyles cités ci-dessus sont droits ou ramifiés et contiennent 1 à 4 atomes de carbone.

Selon l'invention, les produits de formule générale (I) pour lesquels R et Het sont définis comme précédemment à l'exception pour Het de représenter un radical dialcoylaminovinyl-7 naphtyridine-1,8 yle-2 peuvent être préparés par action d'un acide de formule générale:

R—COOH (II)

dans laquelle R est défini comme précédemment, ou d'un dérivé réactif de cet acide, sur une amine de formule générale:

$H_2N$—Het (III)

dans laquelle Het est défini comme précédemment.

Lorsque l'on met en oeuvre l'acide de formule générale (II), on opère en présence d'un agent de condensation peptidique tel qu'un carbodiimide (par exemple le dicyclohexylcarbodiimide) ou le N,N'-

carbonyldiimidazole, ou l'éthoxy-2 éthoxycarbonyl-1 dihydro-1,2 quinoléine, dans un solvant organique tel qu'un éther (par exemple tétrahydrofuranne, dioxanne, glyme, diglyme), un amide (par exemple diméthylformamide), un nitrile (par exemple acétonitrile) ou un solvant chloré (par exemple chlorure de méthylène, dichloroéthane ou chloroforme) à une température comprise entre 0°C et la température de reflux du mélange réactionnel. De préférence on opère à 20°C.

Lorsque l'on met en oeuvre un dérivé réactif de l'acide de formule générale (II) il est possible de faire réagir l'anhydride, un anhydride mixte, un halogénure d'acide ou un ester [qui peut être choisi parmi les esters activés ou non de l'acide de formule générale (II)]. On opère alors soit en milieu organique, éventuellement en présence d'un accepteur d'acide tel qu'une base organique azotée (par exemple une trialcoylamine, une pyridine, le diaza-1,8 bicyclo[5.4.0] undécène-7 ou le diaza-1,5 bicyclo[4.3.0] nonène-5), dans une solvant tel que cité ci-dessus, ou un mélange de ces solvants, à une température comprise entre 0°C et la température de reflux du mélange réactionnel, soit en milieu hydroorganique biphasique en présence d'une base alcaline ou alcalino-terreuse (soude, potasse) ou d'un carbonate ou bicarbonate d'un métal alcalin ou alcalino-terreux à une température comprise entre 0 et 40°C. Il est également possible d'opérer sans solvant à la température de fusion du mélange réactionnel.

Il est entendu que, lorsque, l'acide de formule générale (II) contient un radical hydroxy, ce dernier est préablement protégé, par toute méthode connue qui n'altère pas le reste de la molécule. A titre d'exemple, il est notamment protégé par un radical acétyle qui peut être éliminé par traitement en milieu basique, par exemple par traitement par la potasse éthanolique.

Il est également entendu que lorsque l'acide de formule générale (II) (ou son dérivé réactif), ou l'amine de formule générale (III) portent un substituant contenant une amine secondaire, cette dernière est préablement protégée par toute méthode connue pour la protection des amines sans toucher au reste de la molécule.

On utilise à cet effet tout groupement protecteur facilement éliminable, notamment choisi parmi les groupements habituellement utilisés en chimie peptidique, par exemple les groupements trifluoracétyle, t.butoxycarbonyle, trichloro-2,2,2 éthoxycarbonyle, trityle, benzyle, benzyloxycarbonyle, p.nitrobenzyloxycarbonyle, p.méthoxybenzylloxycarbonyle ou formyle.

Lorsque plusieurs radicaux protecteurs sont présents sur la molécule, l'élimination peut s'effectuer simultanément ou successivement.

A titre d'exemple:

lorsqu'il s'agit d'un radical t.butoxycarbonyle, trityle, p.méthoxybenzyloxycarbonyle ou formyle, l'élimination s'effectue en milieu acide. De préférence on utilise l'acide trifluoracétique à une température comprise entre 0 et 30°C, ou bien on utilise l'acide formique anhydre ou aqueux, ou l'acide paratoluènesulfonique ou méthanesulfonique dans l'acétonitrile, à une température comprise entre 20°C et la température de reflux du mélange réactionnel;

lorsqu'il s'agit d'un radical trifluoracétyle, l'élimination s'effectue par traitement en milieu basique. Par exemple on opère en présence d'un carbonate de métal alcalin en milieu hydro-alcoolique;

lorsqu'il s'agit d'un radical trichloro-2,2,2 éthoxycarbonyle ou p.nitrobenzyloxycarbonyle, l'élimination s'effectue par réduction (par exemple par traitement par le zinc dans l'acide acétique);

lorsqu'il s'agit d'un radical benzyle ou benzyloxycarbonyle, l'élimination s'effectue par hydrogénation catalytique.

Selon l'invention les produits de formule générale (I) dans laquelle R est défini comme précédemment et Het représente un radical acloylsulfinyl-7 naphtyridine-1,8 yle-2 substitué comme défini dans la formule générale (I) peuvent également être préparés par oxydation de l'amide correspondant de formule générale (I) dans laquelle R est défini selon l'addition et Het représente un radical alcoylthio-7 naphtyridine-1,8 yle-2 substitué selon la formule générale (I).

La réaction s'effectue généralement en présence d'un réactif d'oxydation par toute méthode habituellement utilisée pour la préparation d'un sulfoxyde à partir d'un sulfure sans toucher au reste de la molécule. Parmi les réactifs d'oxydation connus, on utilise avantageusement l'eau oxygénée, ou un peracide minérale ou organique par exemple l'acide periodique ou l'acide chloro-3 perbenzoïque.

On opère avantageusement dans un solvant chloré (chlorure de méthylène, chloroforme par exemple) à une température comprise entre 0 et 40°C.

Il est entendu que dans l'alternative où le produit de formule générale (I) dans laquelle Het est un radical alcoylthio-7 naphthyridine-1,8 yle-2 contient des fonctions hydroxy ou amine secondaire, ce dernier peut être mis en oeuvre à l'état protégé à l'issue de sa préparation. La libération des radicaux protecteurs s'effectue alors postérieurement à la réaction d'oxydation par toute méthode qui n'altère pas le reste de la molécule.

Selon l'invention les amides de formule générale (I) dans laquelle R est défini comme précédement et Het est un radical dialcoylaminovinyl-7 napthyridine-1,8, yle-2 peuvent être préparés par action d'un produit de formule générale:

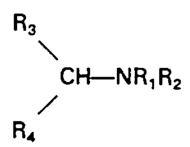

(IV)

dans laquelle $R_1$ et $R_2$ représentent des radicaux alcoyle et $R_3$ et $R_4$ qui sont identiques ou différents représentent soit un groupement alcoyloxy, soit un groupement dialcoylamino, sur un produit de formule générale:

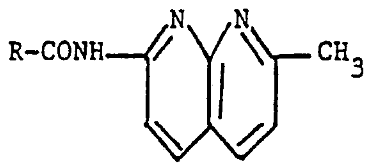

(V)

dans laquelle R a la définition correspondante.

On opère généralement dans un solvant organique tel qu'un amide (diméthylformamide, diméthylacétamide, hexaméthylphosphorotriamide par exemple) ou un nitrile (acétonitrile par exemple), ou dans un mélange de ces solvants, à une température comprise entre 20 et 150°C.

Lorsque l'on utilise un réactif de formule générale (IV) dans laquelle $R_3$ et/ou $R_4$ représentent un radical dialcoylamino, ce radical est choisi de telle manière que l'amine à laquelle il correspond soit plus volatile que $HNR_1R_2$.

Les acides de formule générale (II) peuvent être préparés par application des méthodes décrites ci-après dans les exemples ou par application des méthodes ci-dessous (ou par analogie avec ces méthodes):

H. D. HARTOUGH, The Chemistry of heterocyclic compounds, thiophen and its derivatives, Interscience Publishers Inc. New York, page 363 (1952);

J. W. MASON, The Chemistry of heterocyclic compounds, Pyridazine carboxylic acids, John Wiley and Sons Inc., New York, page 407 (1973);

E. P. OLIVETO, The Chemistry of heterocyclic compounds Pyridinecarboxylic acids, Interscience Publishers Inc., page 179 (1962);

A. A. PETROV et coll., Zhur Obshchei Khim, *26* 1558 (1956), M. E. KUEHNE et coll., Org. Synth. *43,* 22 (1968), S. HUNIG et coll., Chem., Ber., *90,* 238 (1957) ou H. PLIENINGER et coll. Chem. Ber., *94,* 2088 (1961) lorsqu'il s'agit d'un acide cyclohexadiénylcarboxylique.

Les amines de formule générale (III) dans laquelle le symbole Het porte un substituant alcoyloxy ou alcoylthio eventuellement substitués par un radical alcoylamino dialcoylamino (dont les parties alcoyle peuvent former avec l'atome d'azote auquel ils sont rattachés une hétérocycle tel que défini précédemment selon l'addition), ou par un radical N-alcoyl N-alcoyloxycarbonylamino ou dialcoylcarbamoyle, peuvent être préparées à partir de l'amine correspondante dans laquelle le symbole Het est un radical naphtyridine-1,8 yle-2 substitué en position -7 par une atome d'halogène (de préférence un atome de chlore), par action respectivement d'un dérivée hydroxylé ou d'une thiol de formule générales:

R'—OH (VIa) ou R'—SH (VIb)

dans lesquelles R' est un radical alcoyle portant éventuellement un substituant tel que défini ci-dessus, en milieu benzoïque ou par action de l'alcoolate ou du thiolate correspondant.

La réaction s'effectue généralement en présence d'une base forte (soude, potasse, hydroxyde d'ammonium quaternaire, éthylate de sodium par exemple), à une température comprise entre 50 et 150°C, ou bien en présence de l'alcoolate ou de thiolate correspondant (par exemple l'alcoolate de sodium), dans un solvant tel qu'un amide (diméthylformamide par exemple), un éther (tétrahydrofuranne, diméthoxyéthane par exemple), un solvant chloré (chlorobenzène par exemple), un hydrocarbure aromatique (toluène par exemple) ou un oxyde (diméthylsulfoxyde par exemple), ou sans solvant, en présence d'un excès de dérivé hydroxylé ou de thiol, à une température comprise entre 70°C et la température de reflux du mélange réactionnel. Eventuellement on opère en présence d'un agent de catalyse par transfert de phase (par exemple la tris(dioxa-5,6 heptyl)amine ou le chlorure de benzyltriéthylammonium).

Lorsque l'on met en oeuvre l'alcoolate ou le thiolate, celui-ci est obtenu préalablement par action du sodium sur l'alcool de formule générale (VIa) ou le thiol de formule générale (VIb) à une température comprise entre 20 et 80°C, ou par action de l'hydrure de sodium à une température comprise entre 0 et 20°C dans un solvant tel que le diméthylformamide, le diméthoxyéthane, ou le tétrahydrofuranne. Il n'est pas nécessaire d'isoler l'alcoolate obtenu pour le mettre en oeuvre dans la réaction suivante.

L'amine Het-$NH_2$ dans laquelle Het est un radical naphtyridine-1,8 yle-2 substitué en position -7 par un atome d'halogène peut être préparée selon la méthode décrite par S. CARBONI, Gazz. Chim. Italiana, *96,* 1456 (1966).

Les amine de formule générale (III) dans laquelle le symbole Het porte un substituant alcoylsulfinyle lui-même substitué comme défini précédemment, peuvent être préparées par oxydation de l'amine correspondante de formule générale (III) dans laquelle le symbole Het porte un radical alcoylthio substitué.

La réaction s'effectue dans des conditions semblables à celles décrites précédemment pour la préparation d'un amide de formule générale (I) dans laquelle Het porte un substituant alcoylsulfinyl substitué, à partir d'un amide de formule générale (I) dans laquelle Het porte un radical alcoylthio substitué.

Il peut être éventuellement avantageux de protéger la fonction amine préalablement à la réaction. Dans un tel cas on utilise tout groupement protecteur convenable ddont la mise en place et l'élimination

n'altèrent pas le reste de la molécule et qui soit inerte vis-à-vis de la réaction d'oxydation. Par exemple la protection peut être effectuée à l'état de groupements chloracétamido ou phtalimido.

Les produits de formule générale (IV) peuvent être préparés selon les méthodes décrites par H. BREDERECK et coll., Chem. Ber., *101*, 41 (1968) et Angew. Chem. Internat. Ed., *2*, 738 (1963).

Les produits de formule générale (V) peuvent être préparés par analogie avec la méthode de préparation des produits selon l'invention, par action d'un acide de formule générale (II) ou d'un dérivé réactif de cet acide, sur l'amino-2 méthyl-7 naphtyridine-1,8.

L'amino-2 méthyl-7 naphtyridine-1,8 peut être obtenue selon la méthode décrite par E. V. BROWN, J. Org. Chem., *30*, 1607 (1965).

Les nouveaux amides selon la présente invention peuvent être transformés, le cas échéant, en sels d'addition avec les acides.

Comme sels pharmaceutiquement acceptables peuvent être cités les sels d'addition avec les acides minéraux (par exemple chlorhydrates, bromhydrates, sulfates, nitrates, phosphates) ou organiques (par exemple succinates, fumarates, maléates, p.toluène-sulfonates).

Les nouveaux amides selon la présente invention peuvent être purifiés, le cas échéant, par des méthodes physiques telles que la cristallisation ou la chromatographie.

Les produits de formule générale (I) selon l'invention présentent des propriétés pharmacologiques particulièrement intéressantes. Ils manifestent une activité comme anxiolytiques, hypnotiques, anticonvulsivants, antiépileptiques et myorelaxants de bon niveau, mise en évidence dans les tests ci-dessous.

Notamment ils présentent une bonne affinité in vitro pour les sites récepteurs à benzodiazépine à des concentrations comprises entre 10 et des valeurs voisines de 1000 nM selon la technique décrite par J. C. BLANCHARD et L. JULOU, J. of Neurochemistry, *40*, 601 (1983) inspirée des travaux de SQUIRES et BRAESTRUP, Nature, *266*, 732 (1977).

Chez l'animal (souris) ils se sont montrés actifs à des doses comprises entre 10 et 200 mg/kg par voie orale vis-à-vis des convulsions induites par le pentétrazol selon une technique voisine de celle de EVERETT et RICHARDS, J. Pharmacol., *81*, 402 (1944).

Par ailleurs les produits selon l'invention présentent une faible toxicité: leur $DL_{50}$ par voie orale chez la souris est comprise entre 300 mg/kg et des doses supérieures à 900 mg/kg.

D'un intérêt particulier sont les produits de formule générale (I) selon la présente invention pour lesquels

soit le symbole R représente un radical phényle ou phényle substitué par (1 ou 2 atomes de fluor ou par un radical alcoyloxy, alcoylamino ou alcoyloxycarbonylamino en position -4) et le symbole Het représente un radical naphtyridine-1,8 yle-2 substitué en position -7 par un radical alcoyloxy, alcoylthio ou alcoylsulfinyle lui-même substitué [par un radical dialcoylamino (dont les parties alcoyle peuvent former avec l'atome d'azote auquel elles sont rattachées, un hétérocycle à 6 chaiînons contenant éventuellement un autre hétéroatome choisi parmi l'azote, l'oxygène ou le soufre et éventuellement substitué par un radical alcoyle) ou par un radical N-alcoyl N-alcoyloxycarbonyl-amino], ou substitué en position -7 par un radical dialcoylaminovinyle.

soit le symbole R représente un radical alcoylamino-4 phényle ou alcoyloxycarbonylamino-4 phényle et le symbole Het représente un radical naphtyridine-1,8 yle-2 substitué en position -7 par un radical alcoyloxy, étant entendu que les radicaux ou portions alcoyles cités ci-dessus sont droits ou ramifiés et contiennent 1 à 4 atomes de carbone.

Et parmi ces produits, plus spécialement actifs sont les produits de formule générale (I) pour lesquels le symbole R représente un radical phényle substitué par un ou 2 atomes de fluor ou par un radical alcoyloxy en position-4 et le symbole Het représente un radical naphtyridine-1,8 yle-2 substitué en position -7 par un radical alcoyloxy ou alcoylthio contenant 2 ou 3 atomes de carbone, lui-même substitué par un radical dialcoylamino dont les parties alcoyle contiennent 1 ou 2 atomes de carbone:
et notamment les

N-[(diméthylamino-3 propoxy)-7 naphtyridine-1,8 yl-2] méthoxy-4 benzamide,
N-[(diéthylamino-3 propoxy)-7 naphtyridine-1,8 yl-2] méthoxy-4 benzamide,
N-[(diméthylamino-2 éthylthio-7 naphtyridine-1,8 yl-2] méthoxy-4 benzamide,
N-[(diméthylamino-3 propylthio)-7 naphtyridine-1,8 yl-2] méthoxy-4 benzamide,
N-[(diméthylamino-3 propoxy)-7 naphtyridine-1,8 yl-2] difluoro-2,6 benzamide,

Les exemples suivants, donnés à titre non limitatif, illustrent la présente invention.

### Exemple 1

A une solution de 10,2 g d'acide méthoxy-4 benzoïque dans 2000 $cm^3$ de tétrahydrofuranne anhydre, on ajoute 10,9 g de N,N'-carbonyldiimidazole. On observe un dégagement gazeux immédiat. Le mélange est agité pendant 2 heures à une température voisine de 20°C, jusqu'à la fin du dégagement gazeux. On ajoute ensuite 11,6 g d'amino-2 (diméthylamino-2 éthoxy)-7 naphtyridine-1,8 et on chauffe à reflux pendant 25 heures. Le mélange est versé dans 1200 $cm^3$ d'eau distillée, le précipité formé est séparé par filtration, lavé à l'eau, puis séche à l'air et enfin sous pression réduite (0,07 kPa) à 30°C.

Le solide obtenu (5,3 g) est dissous dans 50 $cm^3$ d'éthanol bouillant. Après 15 heures de refroidissement à 4°C, le produit cristallisé est séparé par filtration, lavé par 2 fois 10 $cm^3$ d'éthanol et

séche à 20°C sous pression réduite (0,07 kPa). On obtient 4,5 g de N-[(diméthylamino-2 éthoxy)-7 naphtyridine-1,8 yl-2] méthoxy-4 benzamide fondant à 77—80°C.

L'amino-2 (diméthylamino-2 éthoxy)-7 naphtyridine-1,8 peut être préparée de la façon suivante:

On chauffe pendant 2 heures 30 minutes à 120°C un mélange composé de 89,7 g d'amino-2 chloro-7 naphtyridine-1,8, de 178 g de diméthylamino-2 éthanol et de 65,8 g de potasse en pastilles à 85%. Le mélange obtenu est versé dans 1500 $cm^3$ d'eau distillée. Le produit précipité est éliminé par filtration.

Le filtrat est extrait par 3 fois 300 $cm^3$ de chlorure de méthylène. Les extraits organiques sont lavés par 2 fois 200 $cm^3$ d'au distillée, séchés sur sulfate de magnésium et concentrés à sec à 60°C sous pression réduite (4 kPa).

Le produit obtenu est mis en suspension dans 150 $cm^3$ d'oxyde d'isopropyle. Après 1 heure d'agitation à 25°C, le produit insoluble est filtré puis lavé avec 25 $cm^3$ d'oxyde d'isopropyle et séché à 20°C sous pression réduite (0,07 kPa). On obtient ainsi 45,8 g d'amino-2 (diméthylamino-2 éthoxy)-7 naphtyridine-1,8 fondant à 105°C.

Exemple 2

On opère d'une mainière analogue à celle décrite à l'exemple 1 mais à partir de 10 g d'acide méthoxy-4 benzoïque, de 10,7 g de N,N'-carbonyldiimidazole et de 10,9 g d'amino-2 (diméthylamino-3 propoxy)-7 naphtyridine-1,8, en chauffant 6 heures à reflux. Le mélange réactionnel est versé dans 300 $cm^3$ d'eau et extrait par 3 fois 100 $cm^3$ de chlorure de méthylène. Après concentration à sec des phases organiques, sous pression réduite (4 kPa), le résidu huileux obtenu (21,4 g ) est traité sous agitation avec 10 $cm^3$ d'oxyde d'isopropyle. Le solide cristallisé obtenu (13.9 g; F = environ 80°C) est purifié par chromatographie sur une colonne de 34 mm de diamètre contenant 140 g de silice (0,06—0;,20 mm) en éluant par un mélange (95/5 en volumes) de chlorure de méthylène et de méthanol et en recueillant des fractions de 50 $cm^3$. Aprés concentration à sec des fractions 41 à 99 à 40°C sous pression réduite (4 kPa), on obtient 10,1 g d'une huile épaisse qui est agitée avec 110 $cm^3$ d'oxyde d'isopropyle. Le solide obtenu (4,3 g; F = environ 80°C) est à nouveau purifié par chromatographie "flash" sous 30 kPa sur une colonne de 40 mm de diamètre contenant 130 g de silice (0,04—0,06 mm) en éluant par un mélange (97/3 en volumes) de chlorure de méthylène et de méthanol et en recueillant des fractions de 25 $cm^3$. Après concentration à sec des fractions 10 à 50 à 40°C sous pression réduite (4 kPa) et après séchage à 40°C sous pression réduite (0,07 kPa) on obtient 1 g de N-[(diméthylamino-3 propoxy)-7 naphtyridine-1,8 yl-2] méthoxy-4 benzamide fondant à 130°C.

L'amino-2 (diméthylamino-3 propoxy)-7 naphtyridine-1,8 peut être préparée dde la façon suivante:

Une suspension de 89,75 g d'amino-2 chloro-7 naphtyridine-1,8 dans 236 $cm^3$ de diméthylamino-3 propanol-1 est préchauffée aux environs de 30°C. On introduit ensuite progressivement 65,8 g de potasse en pastilles à 85%. La réaction est exothermique, la température monte jusqu'à 80°C en fin d'addition. On chauffe ensuite encore pendant 30 minutes à 90°C, on laisse refroidir à 60°C puis on verse le mélange réactionnel dans 1500 $cm^3$ d'eau en présence de 500 $cm^3$ de chlorure de méthylène. Les extraits organiques décantés sont lavés par 2 fois 200 $cm^3$ d'eau, séchés sur sulfate de magnésium et concentrés à sec à 40°C sous pression réduite (4 kPa). Le produit obtenu sous forme d'huile est cristallisé par agitation dans 300 $cm^3$ d'hexane. Le solide cristallisé est alors séparé par filtration, lavé à l'hexane et séché à 20°C sous pression réduite (0.07 kPa). On obtient 109 g d'amino-2 (diméthylamino-3-propoxy)-7 naphtyridine-1,8 fondant à 88°C.

Exemple 3

On opère d'une manière analogue à celle décrite à l'exemple 1, mais à partir de 7,1 g d'acide méthoxy-4 benzoïque, de 7,6 g de N,N'-carbonyldiimidazole et de 7,8 g d,amino-2 (diméthylamino-3 méthyl-2 propoxy)-7 naphtyridine-1,8. Le mélange réactionnel est versé dans 500 $cm^3$ d'eau distillée et extrait par 4 fois 100 $cm^3$ de chlorure de méthylène. Les extraits organiques sont lavés par 100 $cm^3$ d'eau distillée, séchés sur sulfate de magnésium et concentrés à sec à 40°C sous pression réduite (4 kPa).

Le produit isolé est dissous dans 50 $cm^3$ d'éthanol puis la solution ainsi obtenue est additionnée de 9,1 $cm^3$ d'une solution éthérée 4N d'acide chlorhydrique. Le précipité formé est séparé par filtration, lavé par 10 $cm^3$ d'éthanol et séché à 40°C sous pression réduite (0,07 kPa).

Le produit obtenu est dissous dans 550 $cm^3$ d'éthanol bouillant. Après 3 heures de refroidissement à 4°C, le solide cristallisé est séparé par filtration, lavé par 10 $cm^3$ d'éthanol et séché à 40°C sous pression réduite (0,07 kPa). On obtient 4,65 g de chlorhydrate de N-[(diméthylamino-3 méthyl-2 propoxy)-7 naphtyridine-1,8 yl-2] méthoxy-4 benzamide, fondant à 260°C.

L'amino-2 (diméthylamino-3 méthyl-2 propoxy)-7 naphtyridine-1,8 peut être préparée de la façon suivante:

On chauffe pendant 3 heures 30 minutes à 120°C un mélange, composé de 21 g d'amino-2 chloro-7 naphtyridine-1,8 de 54,8 g de diméthylamino-3 méthyl-2 propanol et de 15,4 g de potasse en pastilles (à 85%). Le mélange réactionnel est ensuite versé dans 500 $cm^3$ d'eau distillée, et extrait par 3 fois 300 $cm^3$ de chlorure de méthylène. Les extraits organiques sont lavés par 3 fois 200 $cm^3$ d'eau distillée, séchés sur sulfate de magnésium et concentrés à sec à 40°C sous pression réduite (4 kPa).

Le produit obtenu est dissous dans 50 $cm^3$ d'éthanol puis la solution obtenu est additionnée de 82,5 $cm^3$ d'une solution éthérée d'acide chlorhydrique 4N. Le précipité formé est séparé par filtration, lavé par 2 fois 50 $cm^3$ d'éthanol et séché à l'air.

Le produit isolé est dissous dans 200 $cm^3$ d'eau distillée puis la solution obtenue est additionnée de 450

$cm^3$ d'une solution aqueuse à 5% de carbonate de sodium. On extrait par 4 fois 200 $cm^3$ de chlorure de méthylène. Les extraits sont lavés par 6 fois 300 $cm^3$ d'eau distillée séchés sur sulfate de magnésium et concentrés à sec à 40°C sous pression réduite (4 kPa).

Le produit obtenu est dissous dans 500 $cm^3$ d'oxyde d'isopropyle bouillant. Après 18 heures de refroidissement à 4°C, le solide cristallisé est séparé par filtration, lavé par 2 fois 10 $cm^3$ d'oxyde d'isopropyle et séché à 40°C sous pression réduite (0,07 kPa). On obtient 8,5 g d'amino-2 (diméthylamino-3 méthyl-2 propoxy)-7 naphtyridine-1,8 fondant à 108°C.

Exemple 4

On opère d'une manière analogue à celle décrite à l'exemple 1, mais à partir de 4,1 g d'acide méthoxy-4 benzoïque, de 4,3 g de N,N'-carbonyldiimidazole et de 5 g d'amino-2 (diéthylamino-3 propoxy)-7 naphtyridine-1,8. Le mélange réactionnel est ensuite versé dans 500 $cm^3$ d'eau distillée et extrait par 2 fois 100 $cm^3$ de chlorure de méthylène. Les extraits organiques sont lavés par 100 $cm^3$ d'eau distillée, séchés sur sulfate de magnésium et concentrés à sec à 40°C sous pression réduite (4 kPa).

Le produit obtenu est dissous dans 15 $cm^3$ d'éthanol et on ajoute à la solution ainsi obtenue 3,9 $cm^3$ d'une solution éthérée 4N d'acide chlorhydrique. Le précipité blanc formé est séparé par filtrtation, lavé par 10 $cm^3$ d'éthanol et séché à 40°C sous pression réduite (0,07 kPa), puis dissous dans 100 $cm^3$ d'éthanol bouillant. Après 3 heures de refroidissement à 4°C, le solide cristallisé est séparé par filtration, lavé par 10 $cm^3$ d'éthanol et séché à 40°C sous pression réduite (0,07 kPa). On obtient 2,5 g de N-[(diéthylamino-3 propoxy)-7 naphtyridine-1,8 yl-2] méthoxy-4 benzamide fondant à 200°C puis à 240°C.

L'amino-2 (diéthylamino-3 propoxy)-7 naphtyridine-1,8 peut être préparée de la façon suivante:

On chauffe pendant 3 heures à 120°C un mélange composé de 26,9 g d'amino-2 chloro-7 naphtyridine-1,8, de 78 g de diéthylamino-3 propanol-1 et de 19,74 g de potasse en pastilles (à 85%). Le mélange réactionnel est ensuite versé dans 500 $cm^3$ d'eau distillée et extrait avec 500 $cm^3$, puis 3 fois 50 $cm^3$ de chlorure de méthylène. Les extraits organiques sont lavés par 500 $cm^3$ d'eau distillée, séchés sur sulfate de magnésium et concentrés à sec à 40°C sous pression réduite (4 kPa).

Le produit obtenu est dissous dans 100 $cm^3$ d'éthanol et on ajoute à la solution ainsi obtenue, 10 $cm^3$ d'une solution éthérée 3,5N d'acide chlorhydrique. Le précipité formé est séparé par filtration, lavé par 2 fois 50 $cm^3$ d'éthanol et séché à 40°C sous pression réduite (0,07 kPa).

Le produit obtenu est dissous dans 100 $cm^3$ de méthanol bouillant. Après 3 heures de refroidissement à 40°C, le solide cristallisé est séparé par filtration, lavé par 10 $cm^3$ de méthanol et séché à 40°C sous pressioon réduite (0.07 kPa), puis dissous dans 10 $cm^3$ d'eau distillée. A la solution ainsi obtenue on ajoute 55 $cm^3$ d'une solution à 5% de carbonate de sodium. On extrait par 3 fois 60 $cm^3$ de chlorure de méthylène. Les extraits organiques sont lavés par 5 fois 100 $cm^3$ d'eau, séchés sur sulfate de magnésium et concentrés à sec à 40°C sous pression réduite (4 kPa). On obtient ainsi 5 g d'amino-2 (diéthylamino-3 propoxy)-7 naphtyridine-1,8 sous forme d'une huile brune est utilisée telle quelle dans la suite de la synthèse.

Exemple 5

On opère d'une manière analogue à celle décrite à l'exemple 1, mais à partir de 9,9 g d'acide méthoxy-4 benzoïque, de 10,5 g de N.N'-carbonyldiimidazole et de 14,2 g d'amino-2 [(méthyl-4 pipérazinyl-1)-2 éthoxy]-7 naphtyridine-1,8. Le mélange réactionnel est versé dans 600 $cm^3$ d'eau et extrait avec 3 fois 150 $cm^3$ de chlorure de méthylène. Après concentration de phases organiques à sec sous pression réduite (4 kPa), le résidu obtenu est dissous dans 150 $cm^3$ d'éthanol puis la solution obtenue est additionnée de 30,3 $cm^3$ d'une solution éthérée 3,1N d'acide chlorhydrique. Le précipité formé est filtré, lavé par 15 $cm^3$ d'éthanol et séché à 45°C sous pression réduite (0,07 kPa) puis dissous dans 150 $cm^3$ d'eau distillée.

La solution obtenue est alcalinisée à pH 10 avec de la soude 4N et le produit qui précipite est extrait avec 150 $cm^3$ de chlorure de méthylène. Après décantation, la phase aqueuse est extraite à nouveau par 2 fois 150 $cm^3$ de chlorure de méthylène. Les extraits organiques sont lavés avec 2 fois 100 $cm^3$ d'eau, séchés sur sulfate de magnésium et concentrés à sec à 70°C sous pression réduite (4 kPa).

L'huile obtenue est dissoute dans 50 $cm^3$ d'éthanol. On ajoute à la solution ainsi obtenue 3,5 g d'acide fumarique en solution dans 50 $cm^3$ d'éthanol. Après 2 heures à 4°C, le solide cristallisé est séparé par filtration, lavé par 15 $cm^3$ d'éthanol et séché à 40°C sous pression réduite (0,07 kPa). On obtient ainsi 10,2 g de fumarate de N-{[(méthyl-4 pipérazinyl-1)-2 éthoxyl]-7 naphtyridine-1,8 yl-2} méthoxy-4 benzamide, fondant à 175°C.

L'amino-2 [(méthyl-4 pipérazinyl-1)-2 éthoxy]-7 naphtyridine-1,8 peut être préparée de la façon suivante:

On chauffe pendant 6 heures 30 minutes à 105°C un mélange composé de 18 g d'amino-2 chloro-7 naphtyridine-1,8, de 2,88 g d'(hydroxy-2 éthyl)-1 méthyl-4 pipérazine et de 7 g de potasse pulvérisée, à 80%. Le mélange réactionnel est alors coulé dans 800 $cm^3$ d'eau distillée et extrait par 3 fois 200 $cm^3$ de chlorure de méthylène. Les extraits organiques sont lavés à l'eau, séchés sur sulfate de magnésium et concentrés à sec à 70°C sous pression réduite (4 kPa).

Le résidu huileux obtenu est purifié par chromatographie sur une colonne de 32 mm de diamètre contenant 250 g d'alumine neutre, en éluant avec de chlorure de méthylène et en recueillant des fractions de 50 $cm^3$. Après concentration à sec des fractions 3 à 45 à 60°C sous pression réduite (4 kPa), on obtient 10 g d'amino-2 [(méthyl-4 pipérazinyl-1)-2 éthoxy]-7 naphtyridine-1,8 sous forme d'une gomme épaisse.

L'(hydroxy-2 éthyl)-1 méthyl-4 pipérazine peut être préparée selon la méthode décrite par V. ZOTTA et coll., Farmacia, *10*, 605 (1962).

Exemple 6

On opère d'une manière analogue à celle décrite à l'exemple 1, mais à partir de 8 g d'acide méthoxy-4 benzoïque, de 8,5 g de N,N'-carbonyldiimidazole et de 9,6 g d'amino-2 (morpholino-2 éthoxy)-7 naphtyridine-1,8. Le produit obtenu par précipitation dans 500 $cm^3$ d'eau est extrait par 500 $cm^3$ puis 2 fois 250 $cm^3$ de chlorure de méthylène. Après lavage avec 250 $cm^3$ d'eau et séchage sur sulfate de magnésium, les extraits organiques sont concentrés à sec à 50°C sous pression réduite (4 kPa).

L'huile obtenue (17,3 g) est purifiée par chromatographie "flash" sous pression réduite (30 kPa) sur une colonne de 40 mm de diamètre contenant 173 g de silice (0,04—0,06 mm) en éluant par un mélange (98/2 en volumes) de chlorure de méthylène et de méthanol et en recueillant des fractions de 50 $cm^3$.

Le produit obtenu (13,4 g), après concentration à sec des fractions 48 à 95 à 40°C sous pression réduite (4 kPa), est dissous dans 100 $cm^3$ d'éthanol, puis additionné de 9 $cm^3$ d'une solution éthérée 3,8N d'acide chlorhydrique. Le précipité formé est filtré, lavé par 3 fois 20 $cm^3$ d'éthanol, 3 fois 20 $cm^3$ d'oxyde de diéthyle et séché à 50°C sous pression réduite (0.07 kPa). Le produit obtenu est dissous dans 250 $cm^3$ de propanol-1 bouillant. Après 2 heures de refroidissement à 4°C, le solide cristallisé est séparé par filtration, lavé par 3 fois 20 $cm^3$ de propanol-1 et séché à 40°C sous pression réduite (0,07 kPa). On obtient 9 g de chlorhydrate de N-[(morpholino-2 éthoxy)-7 naphtyridine-1,8 yl-2] méthoxy-4 benzamide fondant à 242°C.

L'amino-2 (morpholino-2 éthoxy)-7 naphtyridine-1,8 peut être préparée de la façon suivante:

A une suspension de 16,2 g d'amino-2 chloro-7 naphtyridine-1,8 dans 450 $cm^3$ de chlorobenzène, on ajoute 1,47 g de tris(dioxa-3,6 heptyl) amine, 6,75 g de potasse pulvérisée à 80% et 12 g de morpholino éthanol. Le mélange est porté à 120°C et maintenu pendant 3 heures à cette température. La suspension obtenue après refroidissement est séparée par filtration. Le produit séparé est lavé avec 3 fois 50 $cm^3$ de chlorobenzène et 3 fois 50 $cm^3$ d'eau. Le solide obtenu est redissous dans 400 $cm^3$ de chlorure de méthylène. L'insolube qui subsiste est éliminé par filtration. Le filtrat est séché sur sulfate de magnésium et concetré à sec à 40°C sous pression réduite (4 kPa). Le produit brut obtenu (23,5 g; F = 164°C) est agité en présence de 100 $cm^3$ d'oxyde d'isopropyle, filtré et séché à 30°C sous pression réduite (0,07 kPa). On obtient 13,9 g d'amino-2 (morpholino-2 éthoxy)-7 naphtyridine-1,8 fondant à 164°C.

Exemple 7

On opère d'une manière analogue à celle décrite à l'exemple 1, mais à partir de 21,3 g d'acide méthoxy-4 benzoïque, de 22,7 g de N,N'-carbonyldiimidazole et de 30,6 g d'amino-2 [(N-méthyl N-terbutoxy-carbonyl-amino)-2 éthoxy]-7 naphtyridine-1,8. Le mélange réactionnel est versé dans 1000 $cm^3$ d'eau et extrait par 2 fois 500 $cm^3$ de chlorure de méthylène. Les extraits organiques sont lavés par 500 $cm^3$ d'eau, séchés sur sulfate de magnésium et concentrès à sec à 50°C sous pression réduite (4 kPa).

L'huile obtenue est purifiée par chromatographie sur une colonne de 60 mm de diamètre contenant 400 g de silice (0,06—0,20 mm) en éluant par un mélange (50/50 en volumes) d'acétate d'éthyle et de cyclohexane et en recueillant des fractions de 100 $cm^3$. Après concentration à sec des fractions 6 à 20 à 40°C sous pression réduite (4 kPa), on obtient 35 g d'une huile épaisse. Ce produit est dissous dans 75 $cm^3$ d'éthanol, puis on ajoute 4,35 $cm^3$ d'une solution éthérée 3,8N d'acide chlorhydrique. Le précipité formé est séparé par filtration, lavé avec 3 fois 20 $cm^3$ d'éthanol et séché à 40°C sous pression réduite (0.07 kPa) puis dissous dans 280 $cm^3$ d'acétonitrile bouillant. Aprés 3 heures de refroidissement à 4°C, le produit cristallisé est filtré, lavé par 3 fois 20 $cm^3$ d'acétonitrile, 3 fois 20 $cm^3$ d'oxyde de diéthyle et séché à 50°C sous pression réduite. On obtient 6,5 g de chlorhydrate de N-{[(N-méthyl N-terbutoxycarbonyl-amino)-2 éthoxy]-7 naphtyridine-1,8 yl-2} méthoxy-4 benzamide fondant à 194°C.

L'amino-2 [(N-méthyl N-terbutoxycarbonyl-amino)-2 éthoxy]-7 naphtyridine-1,8 peut être préparée de la façon suivante:

Un mélange constitué de 112 g d'amino-2 chloro-7 naphtyridine-1,8, 1500 $cm^3$ de toluène, 109,4 g de (N-méthyl N-terbutoxycarbonyl-amino)-2 éthanol, 350 g d'une solution aqueuse de soude à 50% en poids et 14,2 g de chlorure de benzyltriéthylammonium est agité pendant 20 heures à une température de 50°C. Le produit insoluble est séparé par filtration et lavé par 3 fois 100 $cm^3$ de toluène et 4 fois 100 $cm^3$ d'eau.

La phase organique du filtrat est séparée par décantation, lavée par 3 fois 300 $cm^3$ d'eau distillée, séchée sur sulfate de magnésium et concentrée à sec à 60°C sous pression réduite (4 kPa). Le résidu obtenu est dissous dans 225 $cm^3$ d'acétonitrile bouillant. Après 2 heures de refroidissement à 4°C, le solide cristallisé est séparé par filtration, lavé par 3 fois 20 $cm^3$ d'acétonitralé et séché à l'air. On obtient 30,6 g d'amino-2 [(N-méthyl N-terbutoxycarbonyl-amino)-2 éthoxy]-7 naphtyridine-1,8 fondant à 170°C.

Le (N-méthyl N-terbutoxycarbonyl-amino)-2 éthanol peut être préparé de la façon suivante:

On ajoute progressivement 70 g de carbonate de sodium à une solution de 50 g de méthylamino-2 éthanol dans 850 $cm^3$ d'eau. La température passe progressivement de 25 à 38°C. On verse ensuite en 5 minutes une solution de 155 g de diterbutyldicarbonate dans 700 $cm^3$ de dioxanne, la réaction est légèrement exothermique. Aprés 15 heures d'agitation aux environs de 20°C, la suspension obtenue est séparée par filtration et lavée par 100 $cm^3$ d'eau. Le filtrat est extrait par 3 fois 500 $cm^3$ de chlorure de méthylène, les extraits organiques réunis sont lavés par 500 $cm^3$ d'eau, séchés sur sulfate de magnésium et concentrés à sec à 60°C sous pression réduite (4 kPa). On obtient 114,7 g de (N-méthyl N-terbutoxycarbonylamino)-2 éthanol sous forme d'une huile.

Spectre infra-rouge, bandes caractéristiques: ($cm^{-1}$) 3625; 3430; 1675; 1395; 1368; 1160; 1050.

Exemple 8

Une solution de 20 g de N-{[(N-méthyl N-terbutoxycarbonylamino)-2 éthoxy]-7 naphtyridine-1,8 yl-2} méthoxy-4 benzamide dans 60 $cm^3$ d'acide trifluoroacétique est agitée pendant 1 heure à une température voisine de 20°C. Le mélange réactionnel est versé dans 100 $cm^3$ d'eau et neutralisé avec 180 $cm^3$ de soude 4N. Le produit insoluble obtenu est séparé par filtration, lavé à l'eau et séché à l'air puis dissous dans 250 $cm^3$ d'acétonitrile bouillant. Après 2 heures de refroidissement à 4°C, le solide cristallisé est séparé par filtration, lavé par 3 fois 20 $cm^3$ d'acétonitrile et séché à 50°C sous pression réduite (0,07 kPa). On obtient 8,7 g de N-[(méthylamino-2 éthoxy)-7 naphtyridine-1,8 yl-2] méthoxy-4 benzamide fondant à 146°C.

Exemple 9

On opère d'une manière analogue à celle décrite à l'exemple 1, mais à partir de 9,3 g d'acide méthoxy-4 benzoïque, de 9,9 g de N,N'-carbonyldiimidiazole et de 10,1 g d'amino-2 (diméthylcarbamoylméthoxy)-7 naphtyridine-1,8. Le mélange réactionnel est versé dans 1250 $cm^3$ d'eau et le précipité obtenu est séparé par filtration, lavé à l'eau et séché à 40°C sous pression réduite (0,07 kPa). On obtient 9,2 g d'un solide fondant à environ 110°C. Le filtrat est extrait par 2 fois 500 $cm^3$ de chlorure de méthylène, lavé à l'eau et concentré à sec à 50°C sous pression réduite (4 kPa) pour donner 4 g d'une gomme. Les deux produits ainsi obtenus sont réunis et purifiés par chromatographie "flash" sous pression réduite (30 kPa) sur une colonne de 40 mm de diamètre contenant 160 g de silice (0,04—0,06 mm) en éluant par un mélange (99/1 en volumes) de chlorure de méthylène et de méthanol et en recueillant des fractions de 50 $cm^3$. Après concentration à sec des fractions 23 à 120 à 40°C sous pression réduite (4 kPa), on obtient 7,3 g d'un solide amorphe. Ce produit est agité avec 150 $cm^3$ d'éther éthylique et le solide cristallisé obtenu est filtré, lavé à l'éther éthylique et séché à l'air. Le solide cristallisé est alors dissous dans 50 $cm^3$ d'un mélange à ébullition d'éthanol et d'eau (50/50 en volumes). Après 2 heures de refroidissement à 4°C, le solide cristallisé est séparé par filtration, lavé par 3 fois 20 $cm^3$ du solvant de cristallisation et séché à 50°C sous pression réduite (0,07 kPa). On obtient 4,3 g de N-[(N-diméthylcarbamoylméthoxy)-7 naphtyridine-1,8 yl-2] méthoxy-4 benzamide fondant à 165°C.

L'amino-2 (diméthylcarbamoylméthoxy)-7 naphtyridine-1,8 peut être préparée de la façon suivante:

On ajoute progressivement 7,5 g de potasse pulvérisée à 80% à une suspension constituée de 12 g d'amino-2 chloro-7 naphtyridine-1,8 et de 25,7 g de N,N-diméthylglycolamide préchauffée à 70°C. La réaction est exothermique. La température est maintenue au voisinage de 100°C pendant la durée de l'addition. Une fois l'addition terminée, on chauffe encore pendant 1 heure 30 minutes à 90°C. Après refroidissement, le mélange réactionnel est versé dasns 200 $cm^3$ d'eau. Le mélange est extrait par 4 fois 200 $cm^3$ de chlorure de méthylène. Les extraits organiques sont lavés par 200 $cm^3$ d'eau, séchés sur sulfate de magnésium et concentrés à sec à 50°C sous pression réduite (4 kPa).

Le produit obtenu est agité avec 100 $cm^3$ d'éther éthylique. Le solide qui cristallise est filtré, lavé à l'éther éthylique et séché à 50°C sous pression réduite (0,07 kPa). On obtient ainsi 10,2 g d'amino-2 (diméthylcarbomoylméthoxy)-7 naphtyridine-1,8 fondant à 182°C.

Le N,N-diméthylglycolamide peut être préparé selon la méthode décrite par W. P. RATCHFORD et coll., J. Org. Chem., *15*, 317 (1950).

Exemple 10

On opère d'une manière analogue à celle décrite à l'exemple 1, mais à partir de 21,1 g d'acide méthoxy-4 benzoïque, de 22,5 g de N,N'-carbonyldiimidazole et de 22 g d'amino-2 (diméthylamino-2 éthylthio)-7 naphtyridine-1,8. Le produit obtenu après traitement du mélange réactionel dans les conditions décrites à l'exemple 1 est séchage est dissous dans 600 $cm^3$ de diméthylformamide et traité par 52 $cm^3$ d'une solution éthérée 3N d'acide chlorhydrique. Le précipité formé est séparé par filtration, lavé pare 3 fois 30 $cm^3$ d'éther éthylique et séché à 40°C sous pression réduite (0,07 kPa).

Le produit obtenu est dissous dans 750 $cm^3$ de méthanol bouillant. Après 3 heures de refroidissement à 4°C, le solide cristallisé est séparé par filtration, lavé avec 30 $cm^3$ de méthanol et séché à 40°C sous pression réduite. On obtient 23,6 g de dichlorhydrate de N-[(diméthylamino-2 éthylthio)-7 naphtyridine-1,8 yl-2] méthoxy-4 benzamide fondant à 260°C avec décomposition.

L'amino-2 (diméthylamino-2 éthylthio)-7 naphtyridine-1,8 peut être préparée de la façon suivante:

On chauffe pendant 2 heures à 90°C un mélange composé de 29,6 g d'amino-2 chloro-7 naphtyridine-1,8, de 48 g de chlorhydrate de diméthylamino-2 éthanethiol, de 43,5 g de potasse en pastilles à 85% et de 300 $cm^3$ de diméthylsulfoxyde. Le mélange réactionnel est ensuite versé dans 400 $cm^3$ d'eau distillée et extrait par 6 fois 250 $cm^3$ de chlorure de méthylène. Les extraits sont lavés par 300 $cm^3$ d'eau distillée, séchés sur sulfate de magnésium et concentrés à sec à 40°C sous pression réduite. Le résidu obtenu est repris par 100 $cm^3$ d'éthanol et le produit insoluble est séparé par filtration, lavé par 2 fois 30 $cm^3$ d'éthanol et séché à 40°C sous pression réduite (0.07 kPa). On obtient 22 g d'amino-2 (diméthylamino-2 éthylthio)-7 naphtyridine-1,8 fondant à 174°C.

Exemple 11

On opère d'une manière analogue à celle décrite à l'exemple 1, mais à partir de 5,32 g d'acide méthoxy-4 benzoïque, de 5,67 g de N,N'-carbonyldiimidazole et de 9 g d'amino-2 (diméthylamino-3 propylthio)-7 naphtyridine-1,8. Le mélange réactionnel est versé dans 1000 $cm^3$ d'eau distillée et extrait par 3 fois 300

$cm^3$ de chlorure de méthylène. Les extraits organiques sont lavés par 200 $cm^3$ d'eau distillée, séchés sur sulfate de magnésium et concentrés à sec à 40°C sous pression réduite (4 kPa).

La pâte marron obtenue (9,32 g) est dissoute dans 50 $cm^3$ d'éthanol puis additionnée de 4,6 $cm^3$ d'une solution éthérée 4,7N d'acide chlorhydrique. Le précipité formé est séparé par filtration, lavé par 10 $cm^3$ d'éther éthylique et séché à 40°C sous pression réduite (0,07 kPa), puis dissous dans 200 $cm^3$ de propanol-1 bouillant. Après 15 heures de refroidissement à 4°C, le solide cristallisé est séparé par filtration, lavé par 2 fois 10 $cm^3$ de propanol-1 et séché à 40°C sous pression réduite (0,07 kPa). On obtient 7.24 g de chlorhydrate de N-[(diméthylamino-3-propylthio)-7 naphtyridine-1,8 yl-2] méthoxy-4 benzamide fondant à 210°C.

L'amino-2 (diméthylamino-3 propylthio)-7 naphtyridine-1,8 peut être préparée de la façon suivante:

On chauffe à 90°C pendant 3 heures 30 minutes un mélange formé de 8 g d'amino-2 chloro-7 naphtyridine-1,8, de 11,4 g de diméthylamino-3 propanethiol, de 6,26 g de potasse en pastilles à 85% et de 87 $cm^3$ de diméthylsulfoxyde. Le mélange réactionnel est ensuite versé dans 1500 $cm^3$ d'eau et extrait par 5 fois 400 $cm^3$ de chlorure de méthylène. Les extraits organiques sont réunis et lavés par 400 $cm^3$ d'eau distillée, séchés sur sulfate de magnésium et concentrés à sec à 40°C sous pression réduite (4 kPa). Le résidu obtenu est traité avec 100 $cm^3$ d'éther éthylique. Le produit insoluble est séparé par filtration, lavé par 10 $cm^3$ d'éther éthylique et séché à 40°C sous pression réduite (0,07 kPa). On obtient 9 g d'amino-2 (diméthylamino-3 propylthio)-7 naphtyridine-1,8 fondant à 164°C.

Exemple 12

On opère d'une manière analogue à celle décrite à l'exemple 1, mais à partir de 5,5 g d'acide benzoïque, de 8,1 g de N,N'-carbonyldiimidazole et de 11,6 g d'amino-2 (diméthylamino-2 éthoxy)-7 naphtyridine-1,8 en chauffant 5 heures à reflux. Le mélange réactionnel est versé dans 1500 $cm^3$ d'eau et extrait par 2 fois 1000 cm d'acétate d'éthyle. Après concentration des phases organiques à sec sous pression réduite (4 kPa), le résidu huileux obtenu (9,2 g) est dissous dans 25 $cm^3$ d'acétate d'éthyle bouillant. Puis après 2 heures de refroidissement à 4°C, le solide cristallisé est séparé par filtration, lavé par 3 $cm^3$ d'acétate d'éthyle et séché à 40°C sous pression réduite (0,07 kPa). On obtient 4,5 g de N-[(diméthylamino-2 éthoxy)-7 naphtyridine-1,8 yl-2] benzamide fondant à 109°C.

Exemple 13

On opère d'une manière analogue à celle décrite à l'exemple 1, mais à partir de 11,3 g d'acide difluoro-2,6 benzoïque, de 12,9 g de N,N'-carbonyldiimidazole et de 11,8 d'amino-2 (diméthylamino-3 propoxy)-7 naphtyridine-1,8. Le produit obtenu par précipitation dans l'eau puis séchage est dissous à ébullition dans 450 $cm^3$ d'acétone. Après 3 heures de refroidissement à 4°C, le solide cristallisé est séparée par filtration, lavé par 10 $cm^3$ d'acétone et séché à 40°C sous pression réduite (0,07 kPa). On obtient 7,2 g de N-[(diméthylamino-3 propoxy)-7 naphtyridine-1,8 yl-2] difluoro-2,6 benzamide fondant à 190°C.

Exemple 14

On opère d'une manière analogue à celle décrite à l'exemple 1, mais à partir de 7 g d'acide N-éthyltrifluoroacétamido-4 benzoïque, de 4,2 g de N,N'-carbonyldiimidazole et de 3,5 g d'amino-2 méthoxy-7 naphtyridine-1,8. Le mélange réactionnel est ensuite versé dans 1000 $cm^3$ d'eau et extrait par 2 fois 200 $cm^3$ de chlorure de méthylène. Les extraits organiques sont séchés sur sulfate de magnésium et concentrés à sec à 40°C sous pression réduite (4 kPa).

Le produit obtenu est dissous dans 20 $cm^3$ de méthanol à reflux puis additionné de 150 $cm^3$ d'oxyde d'isopropyle. Le précipité formé est séparé par filtration, lavé par 2 fois 10 $cm^3$ d'oxyde d'isopropyle et séché 40°C sous pression réduite (0,07 kPa). On obtient 5,1 g de N-(méthoxy-7 naphtyridine-1,8 yl-2) (N'-éthyltrifluoroacétamido)-4 benzamide fondant à 150°C.

On chauffe à 80°C pendant 15 minutes un mélange composé de 5 g de N-(méthoxy-7 naphtyridine-1,8 yl-2) (N'-éthyltrifluoroacétamido)-4 benzamide, de 125 $cm^3$ d'une solution aqueuse à 7% de carbonate de potassium et de 25 $cm^3$ de méthanol. Le précipité formé est séparé par filtration, lavé par 4 fois 20 $cm^3$ d'eau distillée et séché à 40°C sous pression réduite (0,07 kPa).

Ce produit est dissous dans 50 $cm^3$ de méthanol bouillant. Après 2 heures de refroidissement à 20°C, le solide cristallisé est séparé par filtration, lavé par 10 $cm^3$ de méthanol et séché à 40°C sous pression réduite. On obtient 2,2 g de N-(méthoxy naphtyridine-1,8 yl-2) éthylamino-4 benzamide fondant à 134°C puis à 174°C.

L'acide N-éthyltrifluoroacétamido-4 benzoïque peut être préparé de la façon suivante:

On laisse agiter pendant 5 heures à 25°C un mélange composé de 18 g d'acide éthylamino-4 benzoïque, de 20,7 $cm^3$ d'anhydride trifluoroacétique et de 100 $cm^3$ tétrahydrofuranne. Le mélange est ensuite versé dans 200 $cm^3$ d'eau et extrait par 2 fois 200 $cm^3$ de chlorure de méthylène. Les extraits organiques sont lavés par 220 $cm^3$ d'eau distillée, séchés sur sulfate de magnésium et concentrés à sec à 40°C sous pression réduite (4 kPa). Le produit obtenu est purifie par chromatographie sur une colonne de 40 mm de diamètre contenant 320 g de silice (0,04—0,06 mm) en éluant par un mélange (98/2 en volumes) de chlorure de méthylène et de méthanol et en recueillant des fractions de 20 $cm^3$. Après concentration à sec des fractions 2 à 21 à 40°C sous pression réduite (4 kPa) on obtient 7 g d'acide N-éthyltrifluoroacétamido-4 benzoïque fondant à 132°C.

L'acide éthylamino-4 benzoïque peut être préparé de la façon suivante:

On laisse agiter pendant 26 heures à 25°C un mélange composé de 30 g d'acide amino-4 benzoïque, de 32,3 g de borohydrure de sodium et de 550 $cm^3$ d'acide acétique. Le mélange réactionnel est ensuite concentré à sec à 70°C sous pression réduite (4 kPa). Le produit obtenu est traité par 2000 $cm^3$ d'eau distillée et le précipité formé est séparé par filtration, lavé avec 20 $cm^3$ d'eau distillée et séché à l'air. Le produit obtenu est dissous dans 250 $cm^3$ d'acétonitrile bouillant. Après 2 heures de refroidissement à 25°C, le solide cristallisé est séparé par filtration, lavé avec 10 $cm^3$ d'acétonitrile et séché à 40°C sous pression réduite (0,07 kPa). On obtient 18 g d'acide éthylamino-4 benzoïque fondant à 174°C.

Exemple 15

On opère d'une manière analogue à celle décrite à l'exemple 1, mais à partir de 7,3 g d'acide éthoxycarbonylamino-4 benzoïque, de 5,6 g de N,N'-carbonyldiimidazole et de 4,7 g d'amino-2 méthoxy-7 naphtyridine-1,8. Le mélange réactionnel est ensuite versé dans l'eau et le précipité blanc formé est séparé par filtration, lavé par 10 $cm^3$ d'eau et séché à l'air pendant 18 heures.

Le produit obtenu (9,5 g; F = 194°C) est dissous dans 150 $cm^3$ d'acétonitrile bouillant. Après 2 heures de refroidissement à 25°C, le solide cristallisé est séparé par filtration, lavé avec 2 fois 10 $cm^3$ d'acétonitrile et séché à 40°C sous pression réduite. On obtient 6,5 g de N-(méthoxy-7 naphtyridine-1,8 yl-2) éthoxycarbonylamino-4 benzamide fondant à 200°C.

L'acide éthoxycarbonylamino-4 benzoïque peut être préparé de la façon suivante:

On laisse agiter pendant 30 minutes à 5°C un mélange composé de 10 g d'acide amino-4 benzoïque, de 365 $cm^3$ d'une solution aqueuse 1N de bicarbonate de sodium et de 7 $cm^3$ de chloroformiate d'éthyle. On ajoute ensuite au mélange réactionnel 150 $cm^3$ d'acide chlorhydrique 2,5N. Le précipité formé est séparé par filtration, lavé par 3 fois 200 $cm^3$ d'eau et séché à 40°C sous pression réduite (0,07 kPa). On obtient 13,8 g d'acide éthoxycarbonylamino-4 benzoïque fondant à 270°C.

Exemple 16

On opère d'une manière analogue à celle décrite à l'exemple 1, mais à partir de 5,75 g d'acide cyclopropanecarboxylique, de 10,9 g de N,N'-carbonyldiimidazole et de 12,3 g d'amino-2 (diméthylamino-3 propoxy)-7 naphtyridine-1,8. Le mélange réactionnel est versé dans 800 $cm^3$ d'eau et extrait avec 3 fois 200 $cm^3$ de chlorure de méthylène. Les extraits organiques réunis sont lavés par 3 fois 100 $cm^3$ d'eau, séchés sur sulfate de magnésium et concentrés à sec sous pression réduite (4 kPa). L'huile obtenue est dissoute dans 50 $cm^3$ d'éthanol, à 40°C, après traitement au noir, on ajoute au filtrat 150 $cm^3$ d'eau distillée. Après 4 heures de refroidissement à 4°C, le solide obtenu est séparé par filtration, lavé par 20 $cm^3$ d'un mélange d'éthanol et d'eau (1—3 en volume) et séché à 20°C sous pression réduite (0,07 kPa).

Le solide obtenu (12 g) est dissous dans 20 $cm^3$ d'éthanol. On ajoute à la solution ainsi obtenue 4,4 g d'acide fumarique en solution dans 50 $cm^3$ d'éthanol. Après 2 heures à 40°C, le solide cristallisé est séparé par filtration, lavé par 20 $cm^3$ d'éthanol et séché à 40°C sous pression réduite (0,07 kPa). On obtient ainsi 11 g de fumarate de N-[(diméthylamino-3 propoxy)-7 naphtyridine-1,8 yl-2] cyclopropanecarboxamide, fondant à 210°C.

Exemple 17

A une solution de 6 g de N-[(diméthylamino-2 éthylthio)-7 naphtyridine-1,8 yl-2] méthoxy-4 benzamide dans 90 $cm^3$ de chlorure de méthylène à 0°C, on additionne 3,6 g d'acide chloro-3 perbenzoïque. Aprés 2 heures d'agitation à 0°C, le mélange réactionnel est versé dans 200 $cm^3$ de solution de carbonate de sodium à 5%, extrait par 4 fois 100 $cm^3$ de chlorure de méthylène. Les extraits organiques sont lavés avec 100 $cm^3$ d'eau distillée, séchés sur sulfate de magnésium et concentrés à 40°C sous pression réduite (4 kPa).

Le produit est séparé par charomatographie "flash" sous pression réduite (30 kPa) sur 50 g de silice (0,04—0,06 mm) en éluant par un mèlange (95/5 en volumes) de chlorure de méthylène et de méthanol et en recueillant des fractions de 50 $cm^3$. Aprés concentration à sec des fractions 3 à 13 à 40°C sous pression réduite (4 kPa), on obtient 2,3 g d'un solide fondant à 140°C. Ce produit est dissous dans 30 $cm^3$ d'acétonitrile bouillant. Après 1 heure de refroidissement à 5°C, le solide cristallisé est séparé par filtration, lavé avec 2 fois 10 $cm^3$ d'éther éthylique et séché à 40°C sous pression réduite (0.07 kPa). On obtient 2,2 g de N-[(diméthylamino-2 éthylsulfinyl)-7 naphtyridine-1,8 yl-2] méthoxy-4 benzamide fondant à 158°C.

Exemple 18

On ajoute 28 g de terbutoxy bis-(diméthylamino) méthane à une suspension composée de 12 g de N-(méthyl-7 naphtyridine-1,8 yl-2) méthoxy-4 benzamide et de 12 $cm^3$ de diméthylformamide anhydre. Le mélange est agité pendant 3 heures à 130°C. La solution obtenue est versée dans 100 $cm^3$ d'eau puis on extrait avec 200 $cm^3$ de chlorure de méthylène. Les extraits organiques sont lavés par 2 fois 100 $cm^3$ d'eau, séchés sur sulfate de magnésium et concentrés à sec à 50°C sous pression réduite (0,5 kPa). Le résidu amorphe obtenu est dissous dans 100 $cm^3$ de tétrachlorure de carbone bouillant. On précipite un solide par addition lente de 150 $cm^3$ d'oxyde d'isopropyle. Le solide cristallisé obtenu est séparé par filtration, lavé par 30 $cm^3$ d'oxyde d'isopropyle et séché à l'air. La base obtenue (9,2 g; F = environ 110°C) est dissoute dans 150 $cm^3$ d'éthanol puis on ajoute 20 $cm^3$ d'une solution 4,7N d'acide chlorhydrique dans l'éther éthylique. Le précipité obtenu est séparé par filtration, lavé par 3 fois 30 $cm^3$ d'éther éthylique et séché à 25°C sous pression réduite (4 kPa). Le chlorhydrate brut obtenu est purifié par recristallisation dans 160 $cm^3$ d'éthanol.

On obtient ainsi 6 g de N-[(diméthylamino-2 vinyl)-7 naphtyridine-1,8 yl-2] méthoxy-4 benzamide à l'état de chlorhydrate, fondant à 240°C.

La préparation du N-(méthyl-7 naphtyridine-1,8 yl-2) méthoxy-4 benzamide a été décrite précédemment dans la demande de brevet français n° 2 567 887.

La présente invention concerne également les compositions pharmaceutiques qui contiennent les produits de formule générale (I) à l'état pur ou associés à un adjuvant, un diluant et/ou un enrobage compatibles et pharmaceutiquement acceptables. Ces compositions pharmaceutiques peuvent être employées par voie orale, rectale, parentérale ou percutanée.

Comme compositions solides pour administration orale peuvent être utilisés des comprimés des pilules, des poudres (généralement dans des capsules de gélatine) ou des granulés. Dans ces compositions, le produit actif selon l'invention est mélangé à un ou plusieurs diluants inertes, tels que saccharose, lactose ou amidon. Ces compositions peuvent également comprendre des substances autres que les diluants, par exemple un lubrifiant tel que le stéarate de magnésium.

Comme compositions liquides pour administration orale, on peut utiliser des émulsions pharmaceutiquement acceptables, des solutions, des suspensions, des sirops et des élixirs contenant des diluants inertes tels que l'eau ou l'huile de paraffine. Ces compositions peuvent également comprendre des substances autres que les diluants, par exemple des produits mouillants, édulcorants ou aromatisants.

Les compositions selon l'invention pour administration parentérale peuvent être des solutions stériles aqueuses ou non aqueuses, des suspensions ou des émulsions. Comme solvant ou véhicule, on peut employer le propylèneglycol, un polyéthylèneglycol, des huiles végétales, en particulier l'huile d'olive ou des esters organiques injectables, par exemple l'oléate d'éthyle. Ces compositions peuvent également contenir des adjuvants, en particulier des agents mouillants, émulsifiants et dispersants. La stérilisation peut se faire de plusieurs façons, par exemple à l'aide d'une filtre bactériologique, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont des suppositories qui peuvent contenir, outre le produit actif, des excipients tels que le beurre de cacao ou la suppo-cire.

Les compositions pour administration percutanée sont les crèmes, pommades, lotions et liniments, dans lesquels le produit actif est associé à des excipients liquides ou pâteux, de préférence en association avec un véhicule favorisant la migration percutanée.

Les compositions selon l'invention sont particulièrement utiles en thérapeutique humaine pour leur action anxiolytique, hypnotique, anticonvulsivante, antiépileptique et myorelaxante.

En thérapeutique humaine, les doses dépendent de l'effet recherché et de la durée du traitement; elles sont généralement comprises entre 10 et 500 mg par jour par voie orale pour un adulte.

D'une façon générale, le médecin déterminera la posologie qu'il estime la plus appropriée en fonction de l'âge, du poids et de tous les autres facteurs propres au sujet à traiter.

Les exemples suivants, donnés à titre non limitatif, illustrent des compositions selon l'invention.

Exemple A

On prépare selon la technique habituelle des comprimés dosés à 10 mg de produit actif ayant la composition suivante:

| | |
|---|---|
| N-[(diméthylamino-3 propoxy)-7 naphtyridine-1,8 yl-2] méthoxy-4 benzamide | 0,010 g |
| amidon | 0,200 g |
| silice précipitée | 0,036 g |
| stéarate de magnésium | 0,004 g |

Exemple B

On prépare selon la technique habituelle des comprimés dosés à 10 mg de produit actif ayant la composition suivante:

| | |
|---|---|
| dichlorhydrate de N-[(diméthylamino-2 éthylthio)-7 naphtyridine-1,8 yl-2] méthoxy-4 benzamide | 0,012 g |
| amidon | 0,200 g |
| silice précipitée | 0,036 g |
| stéarate de magnésium | 0,004 g |

**Revendications pour les Etats contractants: BE CH DE FR GB IT LI LU NL SE**

1. Un nouvel amide substitué caractérisé en ce qu'il répond à la formule générale:

R—CONH—Het

dans laquelle

soit le symbole R représente un radical cycloalcoyle contenant 3 à 6 atomes de carbone, cyclohexadiényle, phényle, phényle substitué (par 1 ou 2 atomes de fluor ou par un radical hydroxy, ou substitué en position -3 ou -4 par un radical alcoyle ou alcoyloxy, ou en positions -3 et -4 par un radical méthylènedioxy, ou substitué en position -2 ou -3 par un radical dialcoylamino, ou en position -4 par un radical alcoylamino ou alcoyloxycarbonylamino), ou représente un radical hétérocyclyle choisi parmi pyridyle-3, alcoyloxypyridyle-3, thiényle, alcoylthiényle, furyle, tétrahydropyridyle, pyridazinyle et alcoylpyridazinyle et le symbole Het représente un radical naphtyridine-1,8 yle-2 substitué en position -7 par un radical alcoyloxy, alcoylthio ou alcoylsulfinyl lui-même substitué par un radical alcoylamino, dialcoylamino (dont les parties alcoyle peuvent former avec l'atome d'azote auquel elles sont rattachées, un hétérocycle à 5 ou 6 chaînons contenant éventuellement un autre hétéroatome choisi parmi l'azote, l'oxygène ou le soufre et éventuellement substitué par un radical alcoyle) ou par un radical N-alcoyl N-alcoyloxycarbonylamino ou dialcoylcarbamoyle, ou bien substitué en position -7 par un radical dialcoylaminovinyle.

soit le symbole R représente un radical alcoylamino-4 phényle ou alcoyloxycarbonylamino-4 phényle et le symbole Het représente un radical naphtyridine-1,8 yle-2 substitué en position -7 par un radical alcoyloxy ou alcoylthio, étant entendu que les radicaux ou portions alcoyles cités ci-dessus sont droits ou ramifiés et contiennent 1 à 4 atomes de carbone, ainsi que ses sels d'addition avec les acides.

2. Un nouvel amide selon la revendication 1 caractérisé en ce que

soit le symbole R représente un radical phényle ou phényle substitué par 1 ou 2 atomes de fluor ou par un radical alcoyloxy, alcoylamino ou alcoyloxycarbonylamino en position -4 et le symbole Het représente un radical naphtyridine-1,8 yle-2 substitué en position -7 par un radical alcoyloxy, alcoylthio ou alcoylsulfinyle, lui-même substitué [par un radical dialcoylamino (dont les parties alcoyle peuvent former avec l'atome d'azote auquel elles sont rattachées, un hétérocycle à 6 chaînons contenant éventuellement un autre hétéroatome choisi parmi l'azote, l'oxygéne ou le soufre et éventuellement substitué par un radical alcoyle) ou substitué par un radical N-alcoyl N-alcoyloxycarbonyl-amino] on substitué en position -7 par un radical dialcoylaminovinyle.

soit le symbole R représente un radical alcoylamino-4 phényle ou alcoyloxycarbonylamino-4 phényle et le symbole Het représente un radical naphtyridine-1,8 yle-2 substitué en position -7 par un radical alcoyloxy, étant entendu que les radicaux et portions alcoyles cités ci-dessus sont droits ou ramifiés et contiennent 1 à 4 atomes de carbone, ainsi que leurs sels d'addition avec les acides.

3. Un nouvel amide selon la revendication 1 caractérisé en ce que le symbole R représente un radical phényle substitué par un ou 2 atomes de fluor ou par un radical alcoyloxy en position -4 et le symbole Het représente un radical naphtyridine-1,8 yle-2 substitué en position -7 par un radical alcoyloxy ou alcoylthio contenant 2 ou 3 atomes de carbone, lui-même substitué par un radical dialcoylamino dont les parties alcoyle contiennent 1 ou 2 atomes de carbone, ainsi que leurs sels d'addition avec les acides.

4. Le N-[(diméthylamino-3 propoxy)-7 naphtyridine-1,8 yl-2] méthoxy-4 benzamide ainsi que ses sels d'addition avec les acides.

5. Le N-[(diéthylamino-3 propoxy)-7 naphtyridine-1,8 yl-2] méthoxy-4 benzamide ainsi que ses sels d'addition avec les acides.

6. Le N-[(diméthylamino-2 éthylthio)-7 naphtyridine-1,8 yl-2] méthoxy-4 benzamide ainsi que ses sels d'addition avec les acides.

7. Le N-[(diméthylamino-3 propylthio)-7 naphtyridine-1,8 yl-2] méthoxy-4 benzamide ainsi que ses sels d'addition avec les acides.

8. Le N-[(diméthylamino-3 propoxy)-7 naphtyridine-1,8 yl-2] difluoro-2,6 benzamide ainsi que ses sels d'addition avec les acides.

9. Un procédé de préparation d'un amide selon la revendication 1, pour lequel R et Het sont définis comme dans la revendication 1 à l'exception pour Het de représenter un radical dialcoylaminovinyl-7 naphtyridine-1,8 yle-2, caractérisé en ce que l'on fait agir un acide de formule générale:

R—COOH

dans laquelle R est défini comme dans la revendication 1, ou un dérivé réactif de cet acide, sur une amine de formule générale:

$$Het—NH_2$$

dans laquelle Het est défini comme ci-dessus, puis élimine le cas échéant les radicaux protecteurs et transforme éventuellement le produit obtenu en un sel d'addition avec un acide, lorsqu'ils existent.

10. Un procédé selon la revendication 9, caractérisé en ce que l'acide de formule générale:

R—COOH

défini comme dans la revendication 9, dans lequel R porte un radical hydroxy ou le reste d'une amide secondaire, (ou le dérivé réactif de cet acide), est mis en oeuvre à l'état de dérivé protégé.

11. Une procédé selon la revendication 9 caractérisé en ce que l'amine de formule générale:

$$Het—NH_2$$

défini comme dans la revendication 9, dans laquelle Het porte un substituant contenant une amine secondaire, est mise en oeuvre à l'état de dérivé protégé.

12. Un procédé de préparation d'un produit selon la revendication 1, pour lequel R est défini comme dans la revendication 1 et Het est un radical alcoylsulfinyle-7 naphtyridine-1,8 yle-2 substitué par un radical alcoylamino, dialcoylamino (dont les parties alcoyle peuvent former avec l'atome d'azote auquel elles sont rattachées, un hétérocycle à 5 ou 6 chaînons contenant éventuellement un autre hétéroatome choisi parmi l'azote, l'oxygène ou la soufre et éventuellement substitué par un radical alcoyle), ou par un radical N-alcoyl N-alcoyloxycarbonyl-amino ou dialcoylcarbamoyle, caractérisé en ce que l'on oxyde l'amide correspondant selon la revendication 1, pour lequel R est défini comme dans la revendication 1 et Het est un radical alcoylthio-7 naphtyridine-1,8 yle-2 substitué par un radical tel que défini ci-dessus et dont, le cas échéant, les fonctions hydroxy et/ou amine secondaire présentes sur la molécule peuvent être protégées, puis libère le case échéant les radicaux protégés et transforme éventuellement le produit obtenu en un sel d'addition avec un acide, lorsqu'ils existent.

13. Un procédé de préparation d'un produit selon la revendication 1 pour lequel R est défini comme dans la revendication 1 et Het est un radical dialcoylaminovinyl-7 naphtyridine-1,8 yle-2 caractérisé en ce que l'on fait agir un produit de formule générale:

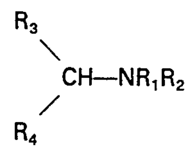

dans laquelle $R_1$ et $R_2$ représentent des radicaux alcoyle et $R_3$ et $R_4$ qui sont identiques ou différentes représentent soit un groupement alcoyloxy, soit un groupement dialcoylamino, dont les parties alcoyle contiennent 1 à 4 atomes de carbone, sur un produit de formule générale:

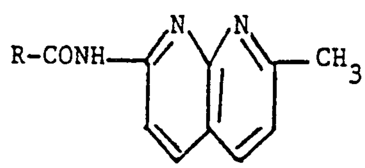

dans laquelle R est défini comme dans la revendication 1, puis transforme éventuellement le produit obtenu en un sel d'addition avec un acide, lorsqu'ils existent.

14. Composition pharmaceutique caractérisé en ce qu'elle contient au moins un produit selon la revendication 1, à l'état pur ou en association avec un ou plusieurs diluants ou adjuvants compatibles et pharmaceutiquement acceptables.

**Revendication pour l'Etat contractant: AT ES GR**

Procédé de préparation d'un nouvel amide substitué caractérisé en ce qu'il répond à la formule générale:

$$R—CONH—Het$$

dans laquelle

soit le symbole R représente un radical cycloalcoyle contenant 3 à 6 atomes de carbone, cyclohexadiényle, phényle, phényle substitué (par 1 ou 2 atomes de fluor ou par un radical hydroxy, ou substitué en position -3 ou -4 par un radical alcoyle ou alcoyloxy, ou en position -3 et -4 par un radical méthylènedioxy, ou substitué en position -2 ou -3 par un radical dialcoylamino ou en position -4 par un radical alcoylamino ou alcoyloxycarbonylamino), ou représente un radical hétérocyclyle choisi parmi pyridyle-3, alcoyloxypyridyle-3, thiényle, alcoylthiényle, furyle, tétrahydropyridyle, pyridazinyle et alcoylpyridazinyle et le symbole Het représente un radical naphtyridine-1,8 yle-2 substitué en position -7 par un radical alcoyloxy, alcoylthio ou alcoylsulfinyle lui-même substitué par un radical alcoylamino, dialcoylamino (dont les parties alcoyle peuvent former avec l'atome d'azote auquel elles sont rattachées, un hétérocycle à 5 ou 6 chaînons contenant éventuellement un autre hétéroatome choisi parmi l'azote, l'oxygène ou le soufre et éventuellement substitué par un radical alcoyle) ou par un radical N-alcoyl N-alcoyloxycarbonyl-amino ou dialcoylcarbamoyle, ou bien substitué en position -7 par un radical dialcoylaminovinyle.

soit le symbole R représente un radical alcoylamino-4 phényle ou alcoyloxycarbonylamino-4 phényle et le symbole Het représente un radical naphtyridine-1,8 yle-2 substitué en position -7 par un radical alcoyloxy ou alcoylthio, étant entendu que les radicaux ou portions alcoyles cités ci-dessus sont droits ou ramifiés et contiennent 1 à 4 atomes de carbone, ainsi que de ses sels d'addition avec les acides, caractérisé en ce que pour préparer un amide pour lequel Het est autre qu'un radical dialcoylaminovinyl-7 naphtyridine-1,8 yle-2, on fait agir un acide de formule générale:

$$R—COOH$$

dans laquelle R est défini comme ci-dessus, ou un dérivé réactif de cet acide, sur une amine de formule générale:

$$Het—NH_2$$

dans laquelle Het est défini comme ci-dessus, puis élimine le cas échéant les radicaux protecteurs, ou bein pour préparer un amide pour lequel Het est un radical alcoylsulfinyle-7 naphtyridine-1,8 yle-2 substitué par un radical alcoylamino, dialcoylamino (dont les parties alcoyle peuvent former avec l'atome d'azote auquel elles sont rattachées, un hétérocycle à 5 ou 6 chaînons contenant éventuellement un autre hétéroatome choisi parmi l'azote, l'oxygène ou la soufre et éventuellement substitué par un radical alcoyle), ou par un radical N-alcoyl N-alcoyloxycarbonyl-amino ou dialcoylcarbamoyle, on oxyde l'amide correspondant de formule générale (I) pour lequel R est défini comme précédemment et Het est un radical alcoylthio-7 naphtyridine-1,8 yle-2 substitué par un radical tel que défini ci-dessus et dont, le cas échéant, les fonctions hydroxy et/ou amine secondaire présentes sur la molécule peuvent être protégées, puis libère le case échéant les radicaux protégés et transforme éventuellement le produit obtenu en un sel d'addition avec un acide, lorsqu'ils existent, ou bien pour préparer un amide pour lequel Het est un radical dialcoylaminovinyl-7 naphtyridine-1,8 yle-2, on fait agir un produit de formule générale:

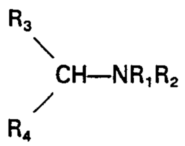

dans laquelle $R_1$ et $R_2$ représentent des radicaux alcoyle et $R_3$ et $R_4$ qui sont identiques ou différentes représentent soit un groupement alcoyloxy, soit un groupement dialcoylamino, dont les parties alcoyle contiennent 1 à 4 atomes de carbone, sur un produit de formule générale:

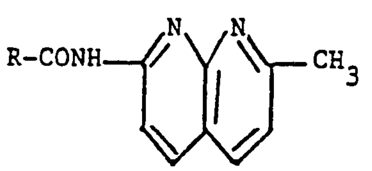

dans laquelle R est défini comme précédemment, puis transforme éventuellement le produit obtenu en un sel d'addition avec un acide, lorsqu'ils existent.

**Patentansprüche für die Vertragsstaaten: BE CH DE FR BE IT LI LU NL SE**

1. Ein neues substituiertes Amid, dadurch gekennzeichnet, daß es der allgemeinen Formel

$$R—CONH—Het$$

entspricht, worin:

entwender das Symbol R einen Cycloalkylrest mit 3 bis 6 Kohlenstoffatomen, Cyclohexadienyl-, substituierten Phenylrest (durch 1 oder 2 Fluoratome oder durch einen Hydroxyrest oder substituiert in 3- oder 4-Stellung durch einen Alkyl- oder Alkyloxyrest oder in den Stellungen 3- und 4 durch einen Methylendioxyrest oder in 2- oder 3-Stellung durch einen Dialkylaminorest, oder in 4-Stellung durch einen Alkylamino- oder Alkyloxycarbonylamino-Rest) oder einen Heterocyclylrest bedeutet, ausgewählt unter Pyridyl-3, Alkyloxypyridyl-3, Thienyl, Alkylthienyl, Furyl, Tetrahydropyridyl, Pyridazinyl und Alkylpyridazinyl, und das Symbol Het einen 1,8-Naphthyridin-2-yl-Rest bedeutet, substituiert in 7-Stellung durch einen Alkyloxy-, Alkylthio- oder Alkylsulfinyl-Rest, der seinerseits durch einen Alkylamino-Dialkylamino-Rest (dessen Alkylteile zusammen mit dem Stickstoffatom, an das sie gebunden sind, einen Heterocyclusrest mit 5 oder 6 Kettengliedern bilden kann, der gegebenenfalls ein weiters Heteroatom enthält, ausgewählt unter Stickstoff, Sauerstoff oder Schwefel und gegebenenfalls durch einen Alkylrest substituiert ist) oder durch einen N-Alkyl-N-alkyloxycarbonylamino- oder Dialkylcarbamoyl-Rest oder auch in 7-Stellung durch einen Dialkylaminovinylrest substituiert ist,

oder das Symbol R einen 4-Alkylaminophenyl- oder 4-Alkyloxycarbonylaminophenyl-Rest bedeutet und das Symbol Het einen 1,8-Naphthyridin-2-yl-Rest bedeutet, der in 7-Stellung durch einen Alkyloxy- oder Alkylthiorest substituiert ist, wobei die vorstehend erwähten Alkylreste oder -teile gerade oder verzweigt sind und 1 bis 4 Kohlenstoffatome enthalten, sowie seine Additionssalze mit Säuren.

2. Neues Amid gemäß Anspruch 1, dadurch gekennzeichnet, daß

entwender das Symbol R einen Phenyl- oder Phenylrest substituiert durch 1 oder 2 Fluoratome oder durch einen Alkyloxy-, Alkylamino- oder Alkyloxy-carbonylamino-Rest in 4-Stellung bedeutet, und das Symbol Het einen 1,8-Naphthyridin-2-yl-Rest, substituiert in 7-Stellung durch einen Alkyloxy-, Alkylthio- oder Alkylsulfinylrest, bedeutet, der seinerseits substituiert ist [durch einen Dialkylaminorest (dessen Alkylteile mit dem Stickstoffatom, an das sie gebunden sind, einen Heterocyclus mit 6 Kettengliedern bilden kann, der gegebenenfalls ein weiteres Heteroatom, ausgewählt unter Stickstoff, Sauerstoff oder Schwefel, enthält und gegebenenfalls substituiert durch einen Alkylrest) oder substituiert durch einen N-Alkyl-N-alkyloxycarbonylaminorest] oder in 7-Stellung durch einen Dialkylaminovinylrest substituiert ist,

oder das Symbol R einen 4-Alkylaminophenyl- oder 4-Alkoxycarbonylaminophenylrest bedeutet und das Symbol Het einen 1,8-Naphthyridin-2-yl-Rest bedeutet, der in 7-Stellung durch einen Alkoxyrest substituiert ist, wobei die vorstehend genannten Alkylreste und -teile gerade oder verzweigt sind und 1 bis 4 Kohlenstoffatome enthalten, sowie ihre Additionssalze mit säuren.

3. Neues Amid gemäß Anspruch 1, dadurch gekennzeichnet, daß das Symbol R einen Phenylrest bedeutet, der durch 1 oder 2 Fluoratome oder durch einen Alkyloxyrest in 4-Stellung substituiert ist, und das Symbol Het einen 1,8-Naphthyridin-2-yl-Rest bedeutet, der in 7-Stellung durch einen Alkyloxy- oder Alkylthiorest mit 2 oder 3 Kohlenstoffatomen substituiert ist, der seinerseits durch einen Dialkylaminorest substituiert ist, dessen Alkylteile 1 oder 2 Kohlenstoffatome enthalten, sowie seine Additionssalze mit Säuren.

4. N-[7-(3-Dimethylamino-propoxy)-1,8-naphthyridin-2-yl]-4-methoxybenzamid sowie seine Additionssalze mit Säuren.

5. N-[7-(3-Diethylamino-propoxy)-1,8-naphthyridin-2-yl]-4-methoxybenzamid, sowie seine Additionssalze mit Säuren.

6. N-[7-(3-Dimethylamino-ethylthio)-1,8-naphthyridin-2-yl]-4-methoxybenzamid, sowie seine Additionssalze mit Säuren.

7. N-[7-(3-Dimethylamino-propylthio)-1,8-naphthyridin-2-yl]-4-methoxybenzamid sowie seine Additionssalze mit Säuren.

8. N-[7-(3-Dimethylamino-propoxy)-1,8-naphthyridin-2-yl]-2,6-difluoro-benzamid sowie seine Additionssalze mit Säuren.

9. Verfahren zur Herstellung eines Amids gemäß Anspruch 1, worin R und Het wie in Anspruch 1 definiert sind mit der Ausnahme, daß Het einen 7-Dialkylaminovinyl-1,8-naphthyridin-2-yl-Rest bedeutet, dadurch gekennzeichnet, daß man eine Säure der allgemeinen Formel

$$R—COOH$$

worin R wie in Anspruch 1 definiert ist, oder ein reaktives Derivat dieser Säure auf ein Amin der allgemeinen Formel

$$Het—NH_2$$

worin Het wie vorstehend definiert ist, einwirken läßt, dann gegebenenfalls die Schutzgruppen entfernt und das erhaltene Produkt gegebenenfalls in ein Additionssalz mit einer Säure, falls sie existieren, überführt.

10. Verfahren gemäß Anspruch 9, dadurch gekennzeichnet, daß die Säure der allgemeinen Formel

$$R—COOH$$

definiert wie in Anspruch 9, worin R einen Hydroxyrest oder den Rest eines sekundären Amids trägt (oder das reaktionsfähige Derivat dieser Säure) im Zustand des geschützten Derivats eingesetzt wird.

11. Verfahren gemäß Anspruch 9, dadurch gekennzeichnet, daß das Amin der allgemeinen Formel

$$Het—NH_2$$

definiert wie in Anspruch 9, worin Het einen ein sekundäres Amin enthaltenden Substituenten trägt, im Zustand des geschützten Derivats eingesetzt wird.

12. Verfahren zur Herstellung eines Produkts gemäß Anspruch 1, wobei R wie in Anspruch 1 definiert ist und Het ein 7-Alkylsulfinyl-1,8-naphthyridin-2-yl-Rest ist, substituiert durch einen Alkylamino-, Dialkylaminorest (dessen Alkylteile mit dem Stickstoffatom, an das sie gebunden sind, einen Heterocyclus mit 5 oder 6 Kettengliedern bilden können, enthaltend gegebenenfalls ein weiteres Heteroatom, ausgewählt unter Stickstoff, Sauerstoff oder Schwefel und gegebenenfalls substituiert durch einen Alkylrest) oder durch einen N-Alkyl-N-alkyloxycarbonylamino- oder Dialkylcarbamoylrest, dadurch gekennzeichnet, daß man das entsprechende Amid gemäß Anspruch 1, worin R wie in Anspruch 1 definiert

ist, und Het ein 7-Alkylthio-1,8-naphthyridin-2-yl-Rest ist, substituiert durch einen Rest wie vorstehend definiert und dessen in dem Molekül gegebenenfalls vorhandene Hydroxy- und/oder sek.-Aminfunktionen geschützt sein können, oxidiert, dann gegebenenfalls die geschützten Reste freisetzt und das erhaltene Produkt gegebenenfalls in ein Additionsalz mit einer Säure, falls solche existieren, überführt.

13. Verfahren zur Herstellung eines Produkts gemäß Anspruch 1, worin R wie in Anspruch 1 definiert ist und Het ein 7-Dialkylaminovinyl-1,8-naphthyridin-2-yl-Rest ist, dadurch gekennzeichnet, daß man ein Produkt der allgemeinen Formel

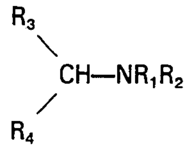

worin $R_1$ und $R_2$ Alkyreste bedeuten und $R_3$ und $R_4$, die identisch oder verschieden sind, entweder eine Alkyloxygruppe oder eine Dialkylaminogruppe bedeuten, deren Alkylteile 1 bis 4 Kohlenstoffatome enthalten, auf ein Produkt der allgemeinen Formel

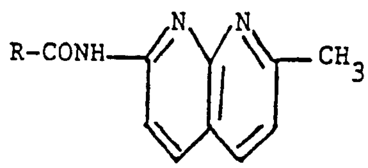

worin R wie in Anspruch 1 definiert ist, einwirken läßt und das erhaltene Produkt gegebenenfalls in ein Additionssalz mit einer Säure, falls solche existieren, überführt.

14. Pharmazeutische Zusammensetzung, dadurch gekennzeichnet, daß sie mindestens ein Produkt gemäß Anspruch 1 in reinem Zustand oder zusammen mit einem oder mehreren verträglichen und pharmazeutisch annehmbaren Verdünnungs- oder Hilfsmitteln enthält.

**Patentanspruch für die Vertragsstaaten: AT ES GR**

Verfahren zur Herstellung eines neuen substituierten Amids, dadurch gekennzeichnet, daß es der allgemeinen Formel:

R—CONH-Het

entspricht, in welcher:

entweder das Symbol R einen Cycloalkylrest mit 3 bis 6 Kohlenstoffatomen, einen Cyclohexadienylrest, einen Phenylrest, einen substituierten Phenylrest (durch 1 oder 2 Fluoratome oder durch einen Hydroxyrest; oder in 3- oder 4-Stellung substituiert durch einen Alkyl- oder Alkyloxyrest, oder in 3- und 4-Stellung durch einen Methylendioxyrest, oder in 2- oder 3-Stellung substituiert durch einen Dialkylaminorest oder in 4-Stellung durch einen Alkylamino- oder Alkyloxycarbonylaminorest) darstellt oder einen Heterocyclylrest, ausgewählt aus 3-Pyridyl, 3-Alkyloxypyridyl, Thienyl, Alkylthienyl, Furyl, Tetrahydropyridyl, Pyridazinyl und Alkylpyridazinyl, darstellt und das Symbol Het einen 1,8-Naphthyridin-2-ylrest, substituiert in 7-Stellung durch einen Alkyloxy-, Alkylthio- oder Alkylsulfinylrest, seinerseits substituiert durch einen Alkylamino-, Dialkylaminorest (deren Alkylteile mit dem Stickstoffatom, an das sie gebunden sind, einen Heterocyclus mit 5 oder 6 Gliedern bilden können, der gegebenenfalls ein weiteres Heteroatom, ausgewählt aus Stickstoff, Sauerstoff oder Schwefel, tragen und gegebenenfalls durch einen Alkylrest substituiert sein kann) oder durch einen N-Alkyl-N-alkyloxycarbonylamino- oder Dialkylcarbamoylrest, oder substituiert in 7-Stellung durch einen Dialkylaminovinylrest, darstellt,

oder das Symbol R einen 4-Alkylaminophenyl- oder 4-Alkyloxycarbonylaminophenylrest darstellt und das Symbol Het einen 1,8-Naphthyridin-2-ylrest, substituiert in 7-Stellung durch einen Alkyloxy- oder Alkylthiorest, darstellt, wobei die oben genannten Alkylreste oder -teile selbstverständlich geradkettig oder verzweigt sind und 1 bis 4 Kohlenstoffatome enthalten, sowie von dessen Säureadditionssalzen, dadurch gekennzeichnet, daß man zur Herstellung eines Amids, worin Het von einem 7-Dialkylaminovinyl-1,8-naphthyridin-2-ylrest verschieden ist, eine Säure der allgemeinen Formel:

R—COOH

in welcher R die obige Bedeutung hat, oder ein reaktionsfähiges Derivat dieser Säure, mit einem Amin der allgemeinen Formel:

Het—$NH_2$

in welcher Het die obige Bedeutung hat, reagieren läßt, dann zutreffendenfalls die Schutzgruppen entfernt, oder daß man zur Herstellung eines Amids, worin Het ein 7-Alkylsulfinyl-1,8-naphthyridin-2-ylrest,

substituiert durch einem Alkylamino-, Dialkylaminorest (deren Alkylteile mit dem Stickstoffatom, an das sie gebunden sind, einen Heterocyclus mit 5 oder 6 Gliedern bilden können, der gegebenenfalls ein weiteres Heteroatom, ausgewählt aus Stickstoff, Sauerstoff oder Schwefel, tragen und gegebenenfalls durch einen Alkylrest substituiert sein kann) oder durch einen N-Alkyl-N-alkyloxycarbonylamino- oder Dialkylcarbamoylrest, ist, das entsprechende Amid der allgemeinen Formel (I), worin R die obige Bedeutung hat und Het ein 7-Alkylthio-1,8-naphthyridin-2-ylrest, substituiert durch einen oben definierten Rest, ist, wobei die am Molekül vorhandenen Hydroxy- und/oder sekundären Aminfunktionen gegebenenfalls geschützt sein können, oxidiert, dann zutreffendenfalls die geschützten Reste freisetzt und das erhaltene Produkt gegebenenfalls in ein Säureadditionssalz umwandelt, falls sie existieren, oder daß man zur Herstellung eines Amids, worin Hat ein 7-Dialkylaminovinyl-1,8-naphthyridin-2-ylrest ist, eine Verbindung der allgemeinen Formel:

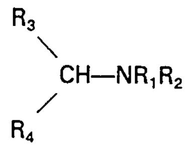

in welcher $R_1$ und $R_2$ Alkylreste darstellen und $R_3$ und $R_4$ gleich oder voneinander verschieden sind und entweder eine Alkyloxygruppe oder eine Dialkylaminogruppe, deren Alkylteile 1 bis 4 Kohlenstoffatome enthalten, darstellt, mit einer Verbindung der allgemeinen Formel:

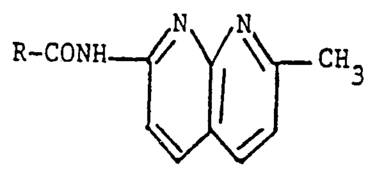

in welcher R die obige Bedeutung hat, reagieren läßt, dann gegebenenfalls das erhaltene Produkt in ein Säureadditionssalz umwandelt, falls sie existieren.

**Claims for the Contracting States: BE CH DE FR GB IT LI LU NL SE**

1. A new substituted amide which is of the general formula:

R—CONH—Het

in which

either the symbol R denotes a cycloalkyl radical containing 3 to 6 carbon atoms, or a cyclohexadienyl, phenyl or substituted phenyl (substituted with 1 or 2 fluorine atoms or with a hydroxyl radical, or substituted at the 3- or 4-position with an alkyl or alkyloxy radical or at the 3- and 4-positions with a methylenedioxy radical, or substituted at the 2- or 3-position with a dialkylamino radical or at the 4-position with an alkylamino or alkyloxycarbonylamino radical) radical, or denotes a heterocyclic radical chosen from 3-pyridyl, 3-alkyloxypyridyl, thienyl, alkylthienyl, furyl, tetrahydropyridyl, pyridazinyl and alkylpyridazinyl, and the symbol Het denotes a 1,8-naphthyridin-2-yl radical substituted at the 7-position with an alkyloxy, alkylthio or alkylsulphinyl radical which is itself substituted with an alkylamino radical or dialkylamino radical (in which the alkyl portions can form, with the nitrogen atom to which they are attached, a 5- or 6-membered heterocyclic system optionally containing another hetero atom chosen from nitrogen, oxygen or sulphur and optionally substituted with an alkyl radical) or with an N-alkyl-N-(alkyloxycarbonyl)amino or dialkylcarbamoyl radical, or alternatively substituted at the 7-position with a dialkylaminovinyl radical,

or the symbol R denotes a 4-(alkylamino)phenyl or 4-(alkyloxycarbonylamino)phenyl radical, and the symbol Het denotes a 1,8-naphthyridin-2-yl radical substituted at the 7-position with an alkyloxy or alkylthio radical, on the understanding that the alkyl radicals or portions mentioned above are linear or branched and contain 1 to 4 carbon atoms, as well as its addition salts with acids.

2. A new amide according to claim 1, in which

either the symbol R denotes a phenyl radical or a phenyl radical substituted with 1 or 2 fluorine atoms or with an alkyloxy, alkylamino or alkyloxycarbonylamino radical at the 4-position, and the symbol Het denotes a 1,8-naphthyridin-2-yl radical substituted at the 7-position with an alkyloxy, alkylthio or alkylsulphinyl radical which is itself substituted [with a dialkylamino radical (in which the alkyl portions can form, with the nitrogen atom to which they are attached, a 6-membered heterocyclic system optionally containing another hetero atom chosen from nitrogen, oxygen or sulphur and optionally substituted with an alkyl radical) or substituted with an N-alkyl-N-(alkyloxycarbonyl)amino radical), or substituted at the 7-position with a dialkylaminovinyl radical,

or the symbol R denotes a 4-(alkylamino)phenyl or 4-(alkyloxycarbonylamino)phenyl radical and the symbol Het denotes a 1,8-naphthyridin-2-yl radical substituted at the 7-position with an alkyloxy radical, on

the understanding that the alkyl radicals and portions mentioned above are linear or branched and contain 1 to 4 carbon atoms, as well as their addition salts with acids.

3. A new amide according to claim 1, in which the symbol R denotes a phenyl radical substituted with one or 2 fluorine atoms or with an alkyloxy radical at the 4-position and the symbol Het denotes a 1,8-naphthyridin-2-yl radical substituted at the 7-position with an alkyloxy or alkylthio radical containing 2 or 3 carbon atoms, the latter radical itself being substituted with a dialkylamino radical in which the alkyl portions contain 1 or 2 carbon atoms, as well as their addition salts with acids.

4. N-[7-(3-Dimethylaminopropoxy)-1,8-naphthyridin-2-yl]-4-methoxybenzamide, as well as its addition salts with acids.

5. N-[7-(3-Diethylaminopropoxy)-1,8-naphthyridin-2-yl]-4-methoxybenzamide, as well as its addition salts with acids.

6. N-[7-(2-Dimethylaminoethylthio)-1,8-naphthyridin-2-yl]-4-methoxybenzamide, as well as its addition salts with acids.

7. N-[7-(3-Dimethylaminopropylthio)-1,8-naphthyridin-2-yl]-4-methoxybenzamide, as well as its addition salts with acids.

8. N-[7-(3-Dimethylaminopropoxy)-1,8-naphthyridin-2-yl]-2,6-difluorobenzamide, as well as its addition salts with acids.

9. A process for preparing an amide according to claim 1, for which R and Het are defined as in claim 1, with the exception of those where Het denotes a 7-dialkylaminovinyl-1,8-naphthyridin-2-yl radical, wherein an acid of general formula:

$$R—COOH$$

in which R is defined as in claim 1, or a reactive derivative of this acid, is reacted with an amine of general formula:

$$Het—NH_2$$

in which Het is defined as above, and, where appropriate, the protective radicals are then removed and the product obtained is optionally converted to an addition salt with an acid, where they exist.

10. A process according to claim 9, wherein the acid of general formula:

$$R—COOH$$

defined as in claim 9, in which R bears a hydroxy radical or a secondary amide residue, (or the reactive derivative of this acid) is employed in the state of a protected derivative.

11. A process according to claim 9, wherein the amine of general formula:

$$Het—NH_2$$

defined as in claim 9, in which Het bears a substituent containing a secondary amine, is employed in the state of a protected derivative.

12. A process for preparing a product according to claim 1, for which R is defined as in claim 1 and Het is a 7-alkylsulphinyl-1,8-naphthyridin-2-yl radical substituted with an alkylamino radical or dialkylamino radical (in which the alkyl portions can form, with the nitrogen atom to which they are attached, a 5- or 6-membered heterocyclic system optionally containing another hetero atom chosen from nitrogen, oxygen or sulphur and optionally substituted with an alkyl radical) or with an N-alkyl-N-(alkoxycarbonyl)amino or dialkylcarbamoyl radical, wherein the corresponding amide according to claim 1, for which R is defined as in claim 1 and Het is a 7-alkylthio-1,8-naphthyridin-2-yl radical substituted with a radical as defined above, and in which, where appropriate, the hydroxy and/or secondary amine groups present on the molecule can be protected, is oxidized and, where appropriate, the protected radicals are liberated and the product obtained is optionally converted to an addition salt with an acid, where they exist.

13. A process for preparing a product according to claim 1 for which R is defined as in claim 1 and Het is a 7-dialkylaminovinyl-1,8-naphthyridin-2-yl radical, wherein a product of general formula:

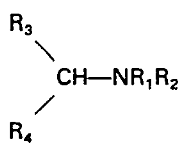

in which $R_1$ and $R_2$ denote alkyl radicals and $R_3$ and $R_4$, which are identical or different, denote either an alkyloxy group, or a dialkylamino group in which the alkyl portions contain 1 to 4 carbon atoms, is reacted with a product of general formula:

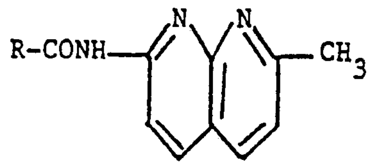

in which R is defined as in claim 1, and the product obtained is optionally converted to an addition salt with an acid, where they exist.

14. A pharmaceutical composition, which contains at least one product according to claim 1, in the pure state or in combination with one or more diluents or adjuvants which are compatible and pharmaceutically acceptable.

**Claim for the Contracting States: AT ES GR**

A process for preparing a new substituted amide which is of the general formula:

R—CONH—Het

in which

either the symbol R denotes a cycloalkyl radical containing 3 to 6 carbon atoms, or a cyclohexadienyl, phenyl or substituted phenyl (substituted with 1 or 2 fluorine atoms or with a hydroxyl radical, or substituted at the 3- or 4-position with an alkyl or alkyloxy radical or at the 3- and 4-positions with a methylenedioxy radical, or substituted at the 2- or 3-position with a dialkylamino radical or at the 4-position with an alkylamino or alkyloxycarbonylamino radical) radical, or denotes a heterocyclic radical chosen from 3-pyridyl, 3-alkyloxypyridyl, thienyl, alkylthienyl, furyl, tetrahydropyridyl, pyridazinyl and alkylpyridazinyl, and the symbol Het denotes a 1,8-naphthyridin-2-yl radical substituted at the 7-position with an alkyloxy, alkylthio or alkylsulphinyl radical which is itself substituted with an alkylamino radical or dialkylamino radical (in which the alkyl portions can form, with the nitrogen atom to which they are attached, a 5- or 6-membered heterocyclic system optionally containing another hetero atom chosen from nitrogen, oxygen or sulphur and optionally substituted with an alkyl radical) or with an N-alkyl-N-(alkyloxycarbonyl)amino or dialkylcarbamoyl radical, or alternatively substituted at the 7-position with a dialkylaminovinyl radical,

or the symbol R denotes a 4-(alkylamino)phenyl or 4-(alkyloxycarbonylamino)phenyl radical, and the symbol Het denotes a 1,8-naphthyridin-2-yl radical substituted at the 7-position with an alkyloxy or alkylthio radical, on the understanding that the alkyl radicals or portions mentioned above are linear or branched and contain 1 to 4 carbon atoms, as well as its addition salts with acids, wherein, to prepare an amide for which Het is other than a 7-dialkylaminovinyl-1,8-naphthyridin-2-yl radical, an acid of general formula:

R—COOH

in which R is defined as above, or a reactive derivative of this acid, is reacted with an amine of general formula:

Het—$NH_2$

in which Het is defined as above, and, where appropriate, the protective radicals are then removed, or alternatively to prepare an amide for which Het is a 7-alkylsulphinyl-1,8-naphthyridin-2-yl radical substituted with an alkylamino radical or dialkylamino radical (in which the alkyl portions can form, with the nitrogen atom to which they are attached, a 5- or 6-membered heterocyclic system optionally containing another hetero atom chosen from nitrogen, oxygen or sulphur and optionally substituted with an alkyl radical) or with an N-alkyl-N-(alkoxycarbonyl)amino or dialkylcarbamoyl radical, the corresponding amide of general formula (I) for which R is defined as above and Het is a 7-alkylthio-1,8-naphthyridin-2-yl radical substituted with a radical as defined above, and in which, where appropriate, the hydroxy and/or secondary amine groups present on the molecule can be protected, is oxidized and, where appropriate, the protected radicals are liberated and the product obtained is optionally converted to an addition salt with an acid, where they exist, or alternatively to prepare an amide for which Het is a 7-dialkylaminovinyl-1,8-naphthyridin-2-yl radical, a product of general formula:

$R_3$
\
CH—$NR_1R_2$
/
$R_4$

in which $R_1$ and $R_2$ denote alkyl radicals and $R_3$ and $R_4$, which are identical or different, denote either an

alkyloxy group, or a dialkylamino group in which the alkyl portions contain 1 to 4 carbon atoms, is reacted with a product of general formula:

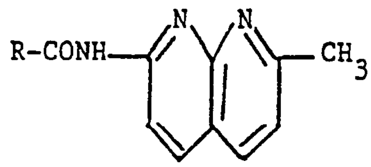

in which R is defined as above, and the product obtained is optionally converted to an addition salt with an acid, where they exist.